# EUROPEAN PATENT SPECIFICATION

(11) **EP 1 539 031 B1**
(45) Date of publication and mention of the grant of the patent: **02.01.2013**
(21) Application number: 03797477.1
(22) Date of filing: 18.09.2003
(51) Int. Cl.: A61F 2/01

(54) **VASCULAR FILTER WITH IMPROVED STRENGTH AND FLEXIBILITY**
GEFÄSSFILTER MIT VERBESSERTER FESTIGKEIT UND FLEXIBILITÄT
FILTRE VASCULAIRE A RESISTANCE ET A SOUPLESSE AMELIOREES

(30) Priority: 19.09.2002 US 412071 P; 09.10.2002 US 417408 P; 01.11.2002 US 423240 P; 26.11.2002 US 304067; 23.04.2003 US 464872 P
(43) Date of publication of application: 15.06.2005
(73) Proprietor: Memory Metal Holland BV, 7534 AT Enschede (NL)
(72) Inventor: Besselink, Petrus, 7534 AT Enschede (NL)
(74) Representative: Crease, Devanand John
(86) International application number: PCT/IB2003/004070
(87) International publication number: WO 2004/026175

(56) References cited:
- EP-A- 1 226 795
- EP-A1- 0 598 635
- WO-A-01/30267
- WO-A-99/44510
- WO-A1-2004/006803
- WO-A1-2004/006804
- US-A- 5 024 671
- US-A- 5 800 525
- US-A- 6 001 100
- US-A1- 2003 023 265
- US-B1- 6 171 338

## Description

This invention relates to medical devices, such as vascular filters to be used in a body lumen, such as a blood vessel, with improved strength and flexibility. A filter according to the invention includes a proximal frame section, a distal section and a flexible thin membrane with perfusion holes of a diameter that allows blood to pass, but prevents the movement of emboli downstream.

Both sections can be collapsed into a small diameter delivery catheter and expanded upon release from this catheter. The membrane has a proximal entrance mouth, which can be expanded, or deployed, substantially to the same size as the body lumen. It is attached to the proximal frame section, which has the function to keep the mouth of the membrane open and prevent the passing of emboli between the body lumen wall and the edge of the filter mouth

In order to have a good flexibility, the membrane is made extremely thin. Normally this would create the risk that the membrane could tear easily, which could cause problems because emboli and pieces of the membrane would then be carried downstream from the filter site.

U.S. Pat. No. 5,885,258 discloses a retrieval basket for catching small particles, made from a slotted tube preferably made of Nitinol, a titanium nickel shape memory alloy. The pattern of the slots allows expansion of the Nitinol basket and by shape setting (heat treatment in the desired unconstrained geometry) this basket is made expandable and collapsible by means of moving it out or into a surrounding delivery tube.

In principle, a distal filter is made of such an expandable frame that defines the shape and enables placement and removal, plus a filter membrane or mesh that does the actual filtering work.

EP 1 226 795 describes a filter device for use in a body lumen. The device has an expandable wire frame around which a wire fibre matrix is spur to form a wire basket.

Sometimes the expandable frame and the mesh are integrated and made from a single material, for example Nitinol, as disclosed in U.S. Pat. No. 6,383,205 or U.S. Published Application No. 2002/0095173. These filters do not have a well-defined and constant size of the holes where the blood flows through, because of the relative movement of the filaments in the mesh. This is a disadvantage, because the size of emboli can be very critical, e.g. in procedures in the carotid arteries. Further the removal of such a filter, accompanied by a reduction of the diameter, may be critical because emboli can be squeezed through the mesh openings with their changing geometry.

A much better control of the particle size is achieved with a separate membrane or filter sheath, which has a well-defined hole pattern with for example holes of 100 microns, attached to a frame that takes care of the correct placement and removal of the filter.

WO 00/67668 discloses a Nitinol basket that forms the framework of the filter, and a separate polymer sheath is attached around this frame. At the proximal side, the sheath has large entrance ports for the blood and at the distal side a series of small holes filters out the emboli. This system, however, has some major disadvantages. First of all, the closed basket construction makes this filter frame rather rigid and therefore it is difficult to be used in tortuous arteries. At a curved part of an artery, it may even not fit well against the artery wall and will thus cause leakage along the outside of the filter.

Another disadvantage of such filters is there is a high risk of squeezing-out the caught debris upon removal, because the struts of the framework force the debris back in the proximal direction, while the volume of the basket frame decreases when the filter is collapsed. Further the construction makes it very difficult to reduce the profile upon placement of the filter. This is very critical, because these filters have to be advanced through critical areas in the artery, where angioplasty and/or stenting are necessary. Of course the catheter that holds this filter should be as small as possible then. In the just described filter miniaturization would be difficult because at a given cross section there is too much material. The metal frame is surrounded by polymer and in the center there is also a guide wire. During angioplasty and stenting, the movements of the guide wire will create further forces that influence the position and shape of the filter, which may cause problems with the proper sealing against the artery wall. This is also the case in strongly curved arteries.

In U.S. Pat. No. 6,348,062, a frame is placed proximal and a distal polymer filter membrane has the shape of a bag, attached to one or more frame loops, forming an entrance mouth for the distal filter bag. Here the bag is made of a very flexible polymer and the hole size is well defined. Upon removal, the frame is closed, thus closing the mouth of the bag and partly preventing the squeezing-out of debris. This is already better than for the full basket design, which was described above, where the storage capacity for debris of the collapsed basket is relatively small. The filter bag is attached to the frame at its proximal end and sometimes to a guide wire at its distal end. Attachment to the guide wire can be advantageous, because some pulling force may prevent bunching of the bag in the delivery catheter.

It may be clear that it is easier to pull a flexible folded bag through a small diameter hole, than to push it through. However, the deformation of the bag material should stay within certain limits.

If the filter is brought into a delivery sheath of small diameter, collapsing the frame and pulling the bag into the delivery sheath causes rather high forces on the connection sites of filter to frame and/or guide wire. While the metal parts of the frame slide easily through such a delivery sheath, the membrane material may have the tendency to stick and in the worst case it may even detach from the frame and tear upon placement or during use, because of too much friction, unlimited expansion, crack propagation or the like.

The connection of the filter bag to the frame is rather rigid, because of the method of direct attachment. Additional flexibility, combined with a high strength attachment spot would also be advantageous.

Methods for making kink resistant reinforced catheters by embedding wire ribbons are described in PCT/US93/01310. There, a mandrel is coated with a thin layer of encapsulating material. Then, a means (e.g. a wire) for reinforcement is deposited around the encapsulating material and eventually a next layer of encapsulating material is coated over the previous layers, including the reinforcement means. Finally the mandrel is removed from the core of the catheter.

Materials for encapsulating are selected from the group consisting of polyesterurethane, polyetherurethane, aliphatic polyurethane, polyimide, polyetherimide, polycarbonate, polysiloxane, hydrophilic polyurethane, polyvinyls, latex and hydroxyethylmethacrylate.

Materials for the reinforcement wire are stainless steel, MP35, Nitinol, tungsten, platinum, Kevlar, nylon, polyester and acrylic. Kevlar is a Dupont product, made of long molecular higly oriented chains, produced from polyparaphenylene terephalamide. It is well known for its high tensile strength and modulus of elasticity.

US-5836962-B1 discloses a medical device with rigid reinforcement fibres embedded in an elongate filament which provides a matrix around the fibres. US-5836962-B1 does not describe a vascular filter comprising a composite reinforced membrane that is attached to a frame and which can be expanded or collapsed.

US-5484424-B1 describes a blood filtration device comprising a catheter of a fibre-reinforced polymer construction and a blood filter. The filter comprises six feet or arms, normally made of metal which constitute a so-called umbrella. The filter does not comprise a composite reinforced membrane that is attached to a frame and which can be expanded or collapsed.

US-5800525-B1 describes a stent-like elastic tubular framework of a fibre-reinforced polymer construction. The framework has a plurality of filter elements which extend from the inner surface in towards the longitudinal axis to trap objects to be filtered. The tubular framework may be covered with a substantially continuous web of a second highly elastic, rubber-like material. US-5800525-B1 mentions that the filtering function of the blood filter is performed by the filter filaments, however, the filter elements are made of the same material as the tubular framework which is a braided nitinol mesh.

In U.S. application Ser. No. 09/537,461 the use of polyethylene with improved tensile properties is described. It is stated that high tenacity, high modulus yarns are used in medical implants and prosthetic devices. Properties and production methods for polyethylene yarns are disclosed.

U.S. Pat. No. 5,578,374 describes very low creep, ultra high modulus, low shrink, high tenacity polyolefin fibers having good strength retention at high temperatures, and methods to produce such fibers. In an example, the production of a poststretched braid, applied in particularly woven fabrics is described.

In U.S. Published Application No. 2001/0034197, oriented fibers are used for reinforcing an endless belt, comprising a woven or non-woven fabric coated with a suitable polymer of a low hardness polyurethane membrane, in this case to make an endless belt for polishing silicon wafers. Examples are mentioned of suitable yarns like meta- or para-aramids such as KEVLAR^{®}, NOMEX^{®} OR TWARON^{®}; PBO or its derivatives; polyetherimide; polyimide; polyetherketone; PEEK; gel-spun UHMW polyethylene (such as DYNEEMA or SPECTRA); or polybenzimidazole; or other yarns commonly used in high-performance fabrics such as those for making aerospace parts. Mixtures or blends of any two or more yarns may be used, as may glass fibers (preferably sized), carbon or ceramic yarns including basalt or other rock fibers, or mixtures of such mineral fibers with synthetic polymer yarns. Any of the above yarns may be blended with organic yarns such as cotton.

This invention relates to systems and methods to fabric then involving angioplasty and/or stenting, where protection against loose embolic material is a major concern, as disclosed in the claims.

Such procedures are performed to remove obstructions or blockages in arteries and thereby alleviate life-threatening conditions. The procedures currently employed result in a fracturing or disintegration of the obstructing material and if the resulting particles, or debris, were permitted to flow downstream within the circulatory system, they would be likely to cause blockages in smaller arteries, or their microscopic branches termed the microcirculation, downstream of the treatment site. The result can be new life-threatening conditions, including stroke.

Various systems and techniques have already been proposed for removing this debris from the circulatory system in order to prevent the debris from causing any harm. These techniques involve temporarily obstruction the artery, at a location downstream of the obstruction, by means of an element such as a balloon, and then suctioning debris and blood from the treatment site. While such techniques can effectively solve the problem stated above, they require that blood flow through the artery be obstructed, causing complete cessation or at least a substantial reduction in blood flow volume, during a time period which can be significant for organ survival for example, the time limit for the brain is measured in seconds and for the heart, in minutes.

Although filters have been used, they suffer from the limitation of either obstructing flow or allowing micro embolism due to fixed pore size. Furthermore, the collected debris can reflux out of the filter when it is closed and lead to embolism. Upon pulling back of a basket/filter with entrapped particles into a delivery catheter, debris particles may be squeezed out of the device, because the volume is strongly reduced. During this pulling back, the filter no longer covers the full cross-section of the artery, so particles that are squeezed out then can freely flow around the outer edge of the filter and move distally through the artery.

The invention also relates to a combined delivery/post-dilatation device for self-expanding stents.

Normally the delivery of self-expanding stents is done with a separate delivery sheath, which is pulled back to release the compressed stent from this sheath and allow it to deploy. If this stent does not deploy to the full size, because the reaction forces of the artery wall and lesion site are too high, it must be further expanded by an additional post-dilatation procedure. Therefore, a separate post-dilatation catheter is needed, that has to be brought into the stented lesion site and then inflated to the full size. This is an extra, time-consuming step in the procedure.

The present invention provides novel medical devices, such as vascular filters, with improved strength and flexibility and methods for their manufacture. These devices have a proximal frame section and a distal section, which can be collapsed into a small diameter delivery catheter and expanded upon release from this catheter. The proximal section is made as a frame of a relatively rigid material compared to the material of the distal section, for example a metal, and the distal section is provided with a flexible thin membrane, with perfusion holes in filter devices, of a diameter that allows blood to pass, but prevents the passage of emboli. The distal filter membrane has a proximal entrance mouth, which has almost the same size as the body lumen of a patient when the filter is deployed. The membrane is attached to the proximal section, which has the function to keep the mouth of the distal filter open and to prevent the passing of emboli between the body lumen wall and the edge of the filter mouth.

In order to have a good flexibility and a minimized crossing profile upon delivery, the membrane is made extremely thin. Tearing of the membrane is prevented by embedding in the filter membrane thin filaments of a material with high strength in the longitudinal direction, but high flexibility upon bending. Such a filter membrane with embedded filaments can have extreme flexibility and elasticity in certain directions, combined with limited deformation, high strength and prevention of crack propagation through the membrane material. Further, the filaments can be attached to the proximal frame section in such a way that the connection points act as hinges and as additional safety for the case that the membrane material might come loose from the frame.

The embedded filaments can include elements that help to give the membrane a desired shape after deployment.

The surface of the membrane filter may be coated with an additional material that improves the properties, for example the biocompatibility, drugs release or any other desired property, which the membrane itself does not offer.

The thus reinforced membranes can also be manufactured without holes for use for parts of catheters, inflatable parts, balloon pumps, replacement of body tissues, repair of body parts and functional parts like artificial valves and membranes, where minimal thickness and/or high strength are required.

Fibers are used not only as reinforcement for the membranes, but are also used as pulling fibers for the extraction the device from a delivery catheter or for retrieval, or retraction, of the device into a removal sheath. The frames can be used in temporary devices like a removable temporary stent, dilator, reamer, a housing for a graft, a valve, a delivery platform for drugs, radiation or gene therapy, or any other device that has to be placed and removed after some time. Applications are not restricted to arteries, but are meant for all body lumens. Placement of the devices discussed herein does not necessarily have to be done by means of a guide wire and accompanying sheath. It can also be done by any displacement member, including the surgeon's hand, stitching, tools, instruments, catheters, balloon or the like.

Further, the invention provides a method for producing devices such as filters by dipping on a removable mold. According to this method, thin filaments of a material with high strength in the longitudinal direction, but high flexibility upon bending, are embedded in the filter membrane. The fibers are preferably less stretchable than the membrane material. The resulting composite membrane can have extreme flexibility and elasticity in certain directions, combined with limited deformation, high strength and prevention of crack propagation through the membrane material. Another function of the embedded filaments is that they help to give the membrane a desired shape after deployment.

The present invention also provides improved devices that prevent escape of debris from the treatment site in a blood vessel, and more specifically prevent embolism, by installing at least one appropriate filter with millipores specific to its use downstream, and possibly one such filter downstream of the treatment site in a blood vessel and manipulating those filters in a manner to assure that any debris created at the treatment site or refluxing from closure of the filters will be removed from the vascular system by physical withdrawal of the filters and/or suction.

For example, an embodiment of the invention may be a multistage, for example two filter, system composed of a first filter to filter the blood flow and a second filter to entrap debris from the first filter.

The invention further relates to a catheter system for delivery of a self-expanding stent with a combined function of delivery from a central sheath and post-dilatation, the system including a catheter having an inflatable outer section that surrounds the sheath at the distal end section of the catheter. The first step in a procedure using this system is the release of the stent by pushing it out of the sheath and pulling back of the catheter over a distance that is equal to at least the length of the stent. Then the catheter is advanced once more until the inflatable section is lined up with the stent again. For post-dilatation the inflatable section is inflated and the lesion plus stent are further expanded.

In one embodiment of the invention, the central lumen within the delivery sheath, where the stent has been pushed out, is reinforced to prevent it from collapsing by the hydraulic pressure of the post-dilatation balloon that surrounds it. Reinforcement of this sheath can be provided by giving the catheter a suitable rigidity at its distal end, for example by giving the catheter an increased thickness at that end. This may make the delivery sheath too rigid, which can be a disadvantage for use in tortuous arteries.

Therefore, the invention makes use of a more flexible delivery sheath that is prevented from collapsing by the use of a separate reinforcement. A pre-dilatation balloon can be lined up with the delivery sheath and inflated until it fills the lumen of this delivery sheath. In this way a concentric arrangement of two balloons, separately inflatable, gives a strong post-dilatation device that is extremely flexible in the deflated state.

A single common guide wire is used to bring the catheters to the lesion site, and the pre-dilatation catheter acts as a guiding means for the stent delivery sheath/post-dilatation balloon. By removal of the pre-dilatation catheter, leaving the inflated delivery catheter in place, a proximal occlusion system is created with a large working channel (the delivery sheath). In combination with a distal occlusion means, e.g. a distal balloon, a closed chamber is created in the artery and this can be reached with a range of instruments for inspection, treatment and flushing/suction purposes.
FIG. 1 is a simplified pictorial view illustrating a first component of a system according to the invention.
FIG. 2 is a simplified pictorial view showing the component for FIG. 1 in an expanded state, associated with a treatment device.
FIG. 3 is view similar to that of FIG. 1 showing the first component and a second component of a system according to the invention.
FIGS. 4A and 4B are simplified pictorial views showing two basic embodiments of the invention.
FIGS. 5, 6 and 7A are cross-sectional elevational views of various alternative embodiments of filter components of a system according to the invention.
FIG. 7B is plan view of the embodiment shown in FIG. 7A.
FIGS. 8, 9 and 10 are simplified pictorial views illustrating specific procedures that may be carried out with a system according to the invention.
FIG. 11 is an elevational view of another embodiment of a filter component of a system according to the invention.
FIG. 12 is a side elevational view of a component of another embodiment of a system according to the invention, including a filter in its folded state.
FIG. 13 is a view similar to that of FIG. 12, showing the filter in its expanded sate.
FIG. 14 is an end view of the component with the filter in the expanded state.
FIG. 15 is a simplified side cross-sectional view showing the other embodiment of a system in a blood vessel with two filters of the type shown in FIGS. 12-14.
FIG. 16 is a view similar to that of FIG. 15 showing a modified form of construction of the system shown in FIG. 15.
FIGS. 17-27 are simplified pictorial views showing successive stages in an angioplasty and stenting procedure using an embodiment of a system according to the invention.

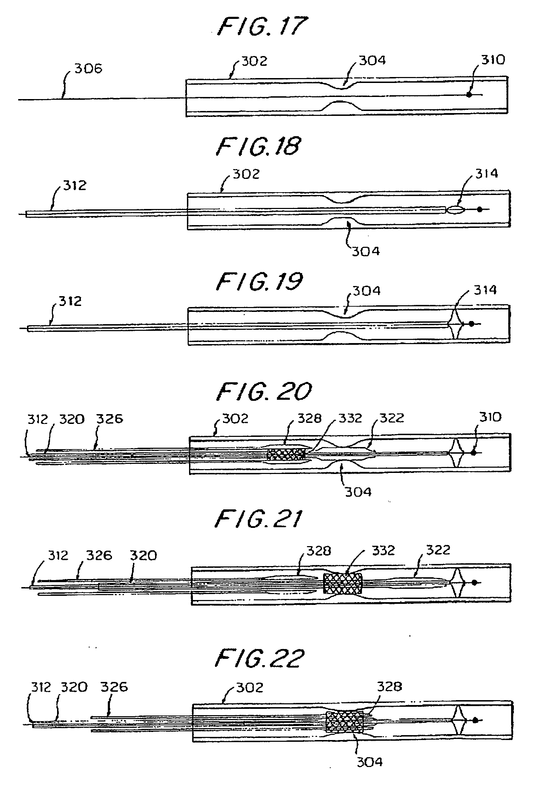

FIG. 17 shows a guide wire brought into an artery with a lesion.
FIG. 18 shows a guiding catheter with a distal protection means, brought across the lesion over the guide wire.
FIG. 19 shows how the distal protection means is deployed until it reaches the artery walls.
FIG. 20 shows a predilatation catheter, which has been advanced over the guiding catheter, in its predilatation position with inflated balloon in the lesion section. Further FIG. 20 shows a delivery sheath with an inflatable distal section, holding a compressed stent, which is advanced over the predilatation balloon catheter.
FIG. 21 shows how the predilatation balloon is deflated and advanced across the lesion site, plus the semi-deployed stent after it has been delivered in the lesion area.
In FIG. 22 the two balloons are lined up and brought in the stent.

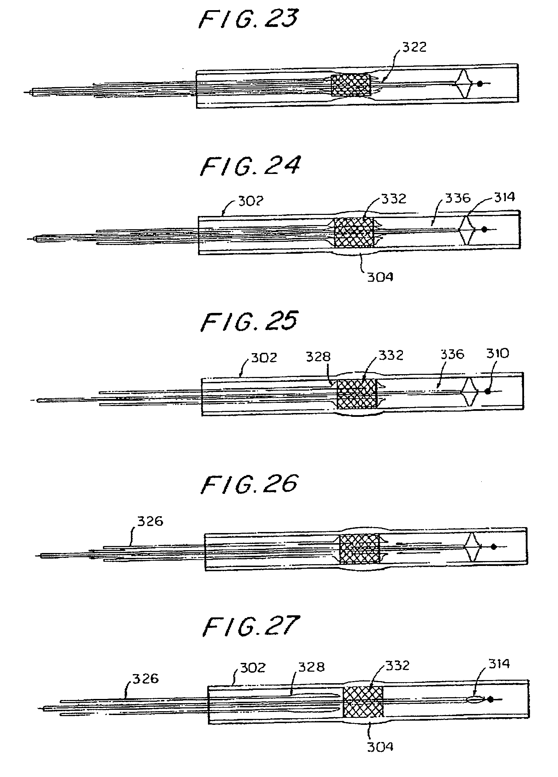

In FIG. 23 the predilatation balloon is inflated to create a support for the inflatable delivery sheath.
In FIG. 24 the inflatable delivery sheath is inflated to perform the final angioplasty and to reach full deployment of the stent.
In FIG. 25 the predilatation balloon catheter is removed from the patient's body while the inflated sheath is still in place.
In FIG. 26 the chamber in the artery between distal protection means and inflated sheath is flushed to remove or catch all debris.
In FIG. 27 the sheath is deflated and the distal protection means is collapsed, thus enabling removal from the artery, leaving only the stent in place.

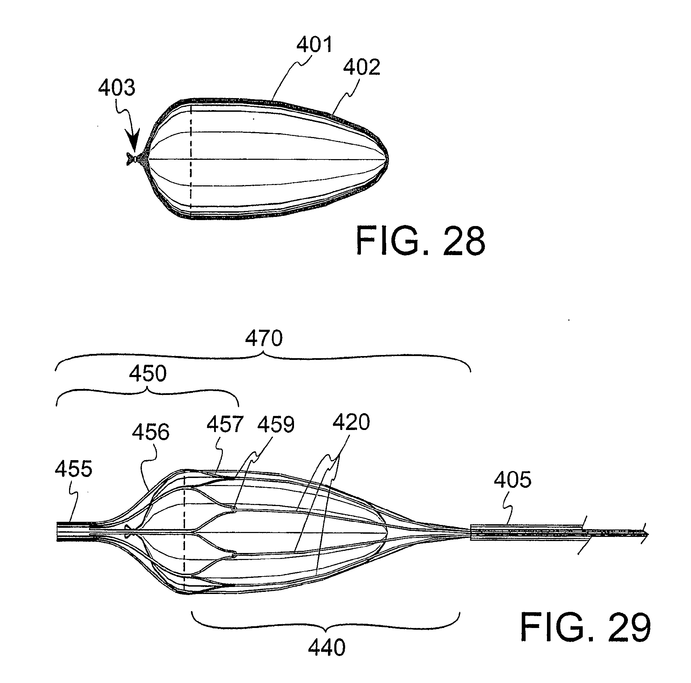

FIGS. 28-31 are side elevational views showing four stages in the fabrication of an embodiment of a filter according to the present invention.

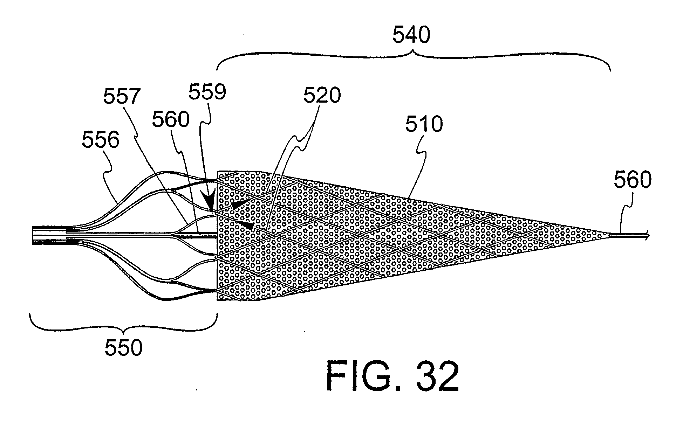

FIG. 32 is an elevational view showing another embodiment of a filter according to the present invention.

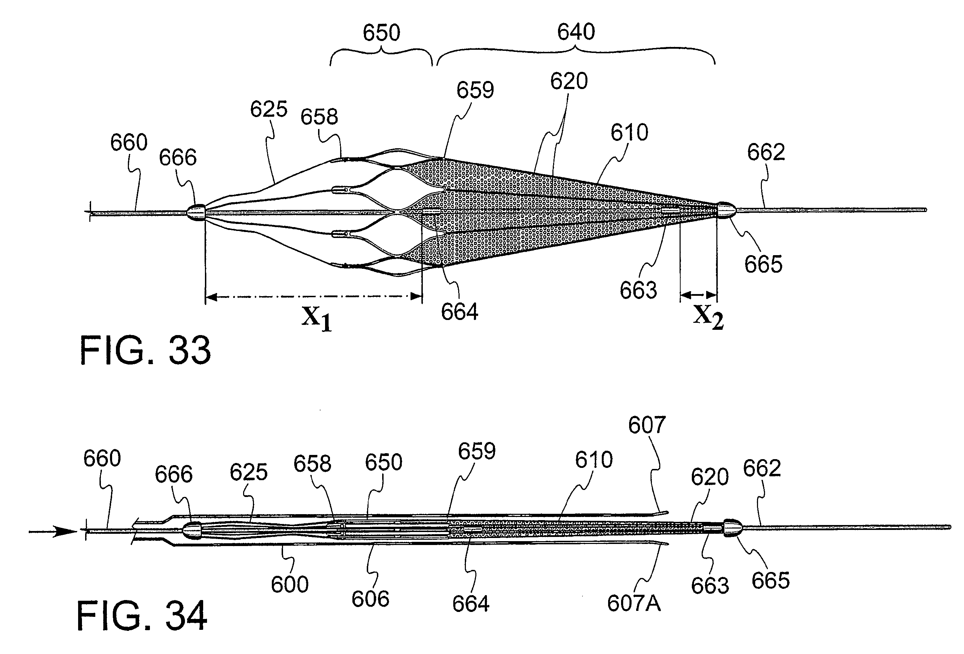

FIG. 33 is a side elevational view showing another embodiment of a filter according to the present invention in an expanded state.
FIG. 34 shows the filter of FIG. 33 in a compressed state while being inserted to a desired location with a delivery sheath.

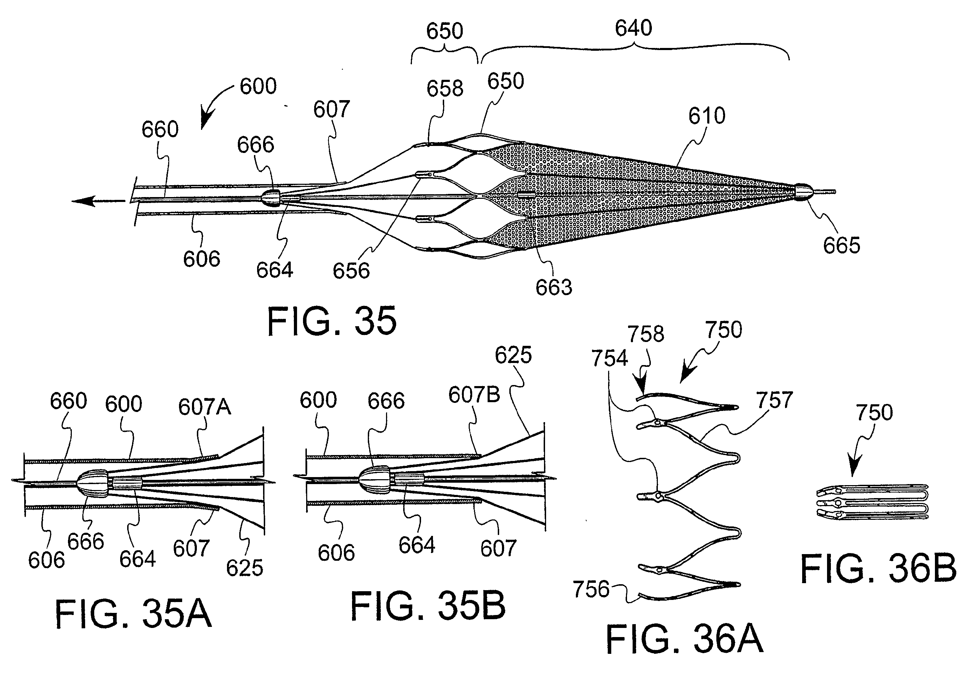

FIG. 35 shows the filter of FIG. 33 being withdrawn back into the sheath.
FIG. 35a is a detail view of a portion of the embodiment of FIGS. 33-35.
FIG. 35b is a detail view similar to that of FIG. 35a, showing a modified version of a component of the embodiment of FIGS. 33-35.
FIGS. 36a and 36b are detail views of a modified form of construction of a portion of the embodiment of FIGS. 33-35.

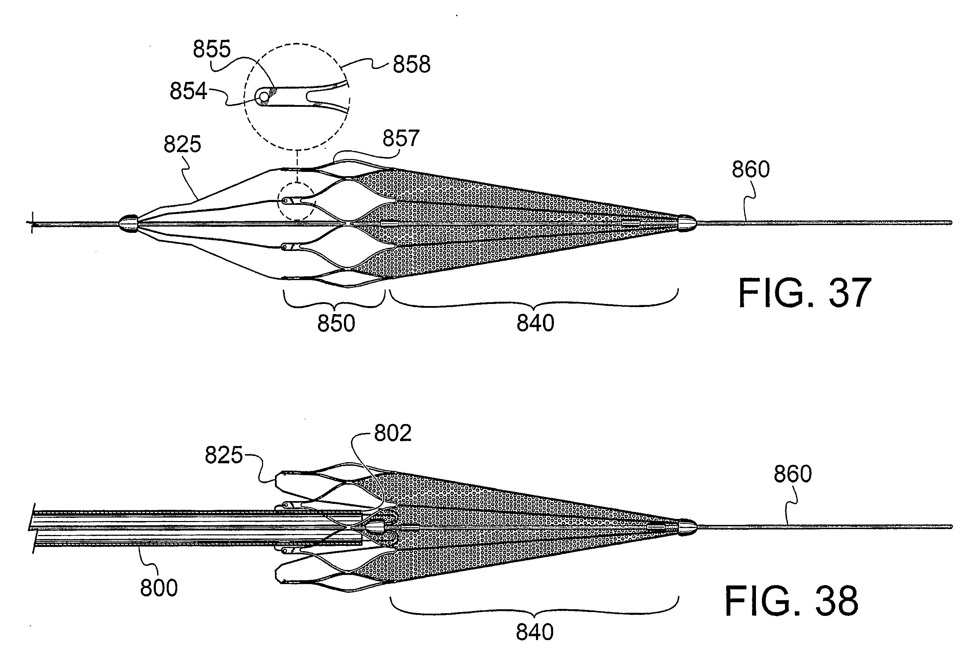

FIG. 37 is a side elevational view showing a modified version of the embodiment shown in FIGS. 33-35, and includes an inset illustrating the modification to a larger scale.
FIG. 38 is a side elevational view showing the filter of FIG. 37 in a further possible operating stage.

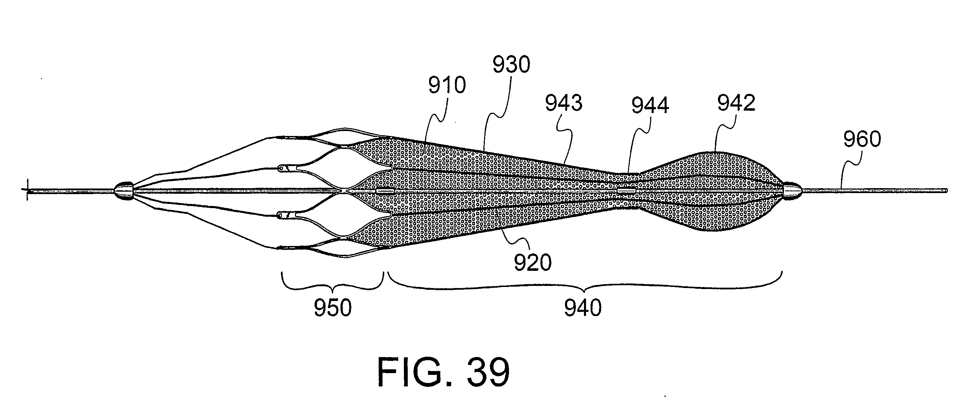

FIG. 39 is a side elevational view showing another embodiment of a filter according to the present invention.

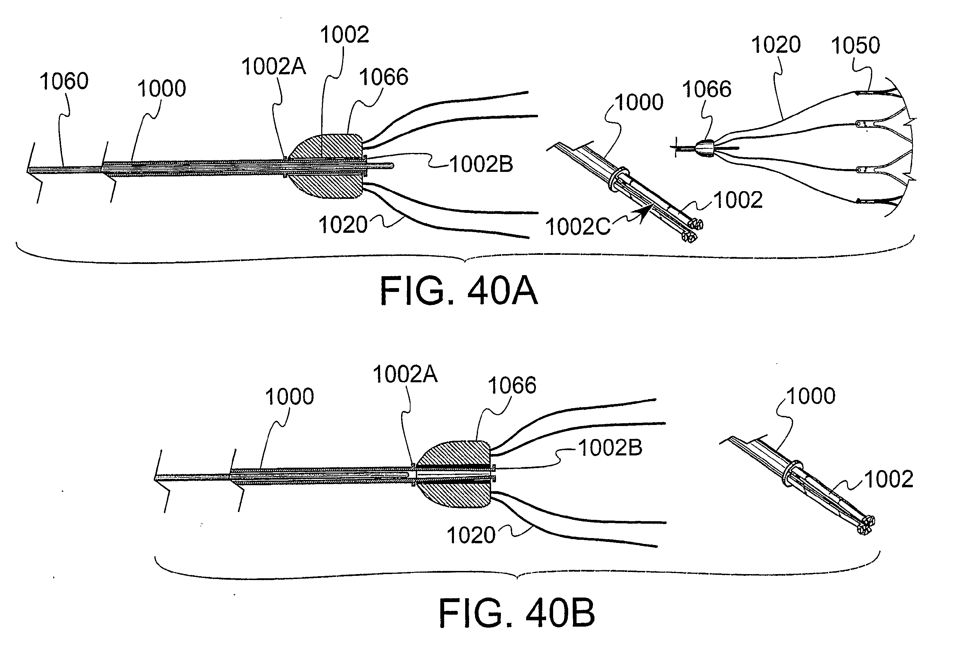

FIGS. 40a through 40c show a delivery system that enables a device of the present invention to be permanently placed within a body lumen.

The invention provides a novel method and a system to confine and remove debris from a blood vessel, thereby preventing embolism in the vascular system.

A first step of one embodiment of a method according to the invention includes positioning a first particle filter in the blood vessel downstream of the treatment site.

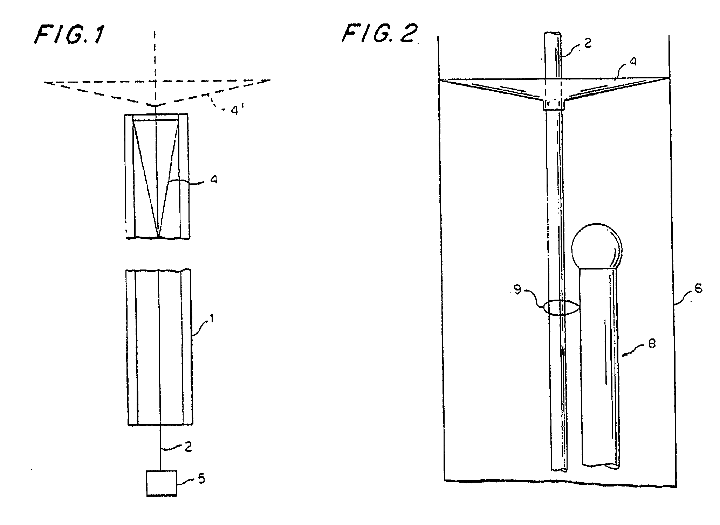

FIG. 1 is a cross-sectional elevational view of a first unit of a protective system according to the invention for carrying out the first step. This unit is composed of a sheath 1, a hollow guide wire 2 and a distal particle filter 4.

Filter 4 may have any shape, for example a conical shape, as shown, and is constructed to be radially expansible from a radially compressed state, shown in solid lines, to a radially expanded state, shown in broken lines at 4'. Preferably, at least one part of filter 4 is made of a resiliently deformable material that autonomously assumes the radially expanded state shown at 4' when unconstrained. Filter 4 may be shaped using appropriate shape setting procedures to open with a flared top portion made from highly elastic material such as the memory metal nitinol.

Sheath 1 serves to hold filter 4 in the radially compressed state during transport of filter 4 to and from the treatment site.

Filter 4 has a tip, or apex, that is fixed to guide wire 2. Guide wire 2 extends from a proximal end that will always be outside of the patient's body and accessible to the physician to a distal end that extends past the apex.

Guide wire 2 is preferably a hollow tube whose distal end is, according to the invention, used as a pressure sensor in communication with a pressure monitoring device 5 connected to the proximal end of guide wire 2. Device 5 is exposed to, and senses, via the longitudinal passage, or bore, in tube 2, the pressure adjacent to the distal end of guide wire 2.

Preferably, monitoring device 5 is removably fastened to the proximal end of guide wire 2. Device 5 would be removed, for example, when guide wire 2 is to be used to guide some other component of the device into the blood vessel after insertion of the first unit into a blood vessel, as will be described in greater detail below.

According to one practical embodiment of the invention, sheath 1 has an outside diameter of 1 to 1.5 mm and wire 2 has an outside diameter of 0.014-0.018 inch (approximately 0.5 mm) and is sized so that during insertion it will not disturb the obstruction that is to be removed. Filter 4 can be dimensioned to expand to an outer diameter of more than 1 mm, and preferably more than 10 mm. This dimension will be selected to be approximately as large as the diameter of the vessel to be treated.

Prior to insertion into a blood vessel filter 4 is arranged in sheath 1 as shown in FIG. 1. Then, in a conventional preliminary step, the blood vessel wall is punctured by a hollow needle, a preliminary guide wire (not shown) is introduced into the blood vessel through the needle, the needle is withdrawn, the opening in the blood vessel is dilated and a guiding catheter (not shown) is passed over the preliminary guide wire into the blood vessel to be treated. The distal, or leading, end of the guiding catheter is brought to an appropriate point ahead of an obstruction to be treated and the preliminary guide wire is withdrawn. Then, guide wire 2 and sheath 1, with filter 4 in place, are introduced into the blood vessel in the direction of blood flow, in a conventional manner through the guiding catheter, until filter 4 is at the desired location in the vessel, usually downstream of the obstruction to be treated. Introduction through the guiding catheter facilitates accurate passage of the filter 4 and sheath 1 by preventing buckling and permitting easier positioning, as well as reducing the risk of dislodging clot particles from the obstruction, which is typically plaque. Then, the operator holds wire 2 stationary and retracts sheath 1, which is long enough to be accessible to the operator outside the body, until sheath 1 moves clear of filter 4, which can then expand to take on the configuration shown at 4'. Sheath 1 can then be fully withdrawn from the vessel. Whenever required, the proximal end of sheath 1 can be clamped shut, usually during withdrawal.

A second step of a method according to the invention involves performance of the desired medical treatment in the region upstream of filter 4, which region, as shown in FIG. 2, is below filter 4. Such a treatment can be for the purpose of removing an obstruction in a blood vessel 6, and this can involve any known angioplasty procedure or any known obstruction disintegration or observation (viewing) procedure employing ultrasound, laser radiation, stent placement, etc., or any mechanical cutting procedure, etc. The device for performing this function can be guided to the site by being advanced along guide wire 2.

For example, this device can be an ultrasonic device as disclosed in U.S. Pat. No. 4,870,953. This device has an output end 8 provided with a bulbous tip that applies ultrasonic vibrations to obstruction material, such as plaque or clot. Output end 8 may be guided to the site of the obstruction in any conventional manner over wire 2, however this can be assisted by providing output end 8 with a ring, or loop, 9 that is fitted around guide wire 2 before output end 8 is introduced into blood vessel 6.

After the device has been brought to the treatment site, it is operated to perform the desired treatment, in this case disintegration of plaque or clot, commonly predilation, stenting and stent dilatation. After the treatment has been performed, the treatment device is withdrawn from the blood vessel.

A third step of a method according to the invention includes positioning a second particle filter in the blood vessel upstream of first filter 4 and preferably upstream of the treatment site. This is accomplished by sliding guide wire 2 through an orifice in a second filter 14, to be described below, adjacent to a guide wire 12 that carries the second filter.

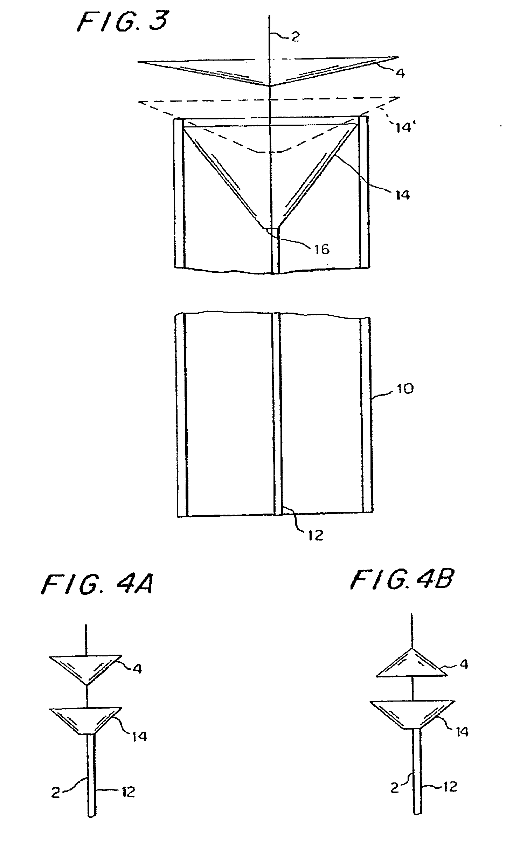

FIG. 3 is cross-sectional elevational view of a second unit of the protective system according to the invention for carrying out the third step.

This second unit is composed of a second tube, or sheath, 10, a second guide wire 12 and a proximal particle filter 14. Sheath 10 may have a diameter of the order of 3 mm. At the time this unit is inserted into the blood vessel, filter 4 remains in place in the blood vessel, in the expanded state as shown at 4' in FIG. 1, as does hollow guide wire 2.

Proximal filter 14 has an apex provided with a ring 16 through which guide wire 2 is inserted when the second unit is still located outside of the patient's body, in order to guide the second unit into the blood vessel up to the treatment site. Second guide wire 12 is secured to ring 16.

Prior to introduction into the patient's body, filter 14 is installed in sheath 10 in the manner illustrated in FIG. 3. The second unit is then placed over guide wire 2 and advanced into the blood vessel to the desired location.

After the second unit has been brought to the desired location, proximal filter 14 is held stationary by holding stationary the end of guide wire 12 that is outside of the patient's body, while retracting sheath 10. When filter 14 is clear of the distal end of sheath 10, filter 14 expands radially into the configuration shown at 14' to engage filter 4. This step is completed when filter 14 is fully radially expanded.

Because of the porous nature of filters 4 and 14, a reasonable volume of blood flow can be maintained in the blood vessel when the filters are deployed.

Prior to introduction of filter 14, any debris produced by the treatment performed in the second step will be conveyed by blood flowing to and through radially expanded filter 4, where the debris will tend to remain. During and after introduction of filter 14 and expansion of filter 14 into the configuration shown at 14', suction may be applied to the region between the filters through sheath 10. This will help to assure that the debris remains trapped between the two filters.

Then, in a fourth step, debris is removed from blood vessel 6 by pulling wire 2 to move filter 4 toward, and into contact with, filter 14, then retracting both filters into sheath 10 by pulling the guide wires 2 and 12, thus withdrawing the assembly of filters 4 and 14 into sheath 10. Sheath 10 with enclosed filters is then withdrawn through the guiding catheter (not shown), which is subsequently removed from the blood vessel using standard procedures. These operations are performed by pulling on guide wire 2 at its proximal end, located outside of the patient's body, while initially holding guide wire 12 stationary until filter 4, comes to nest within filter 14. Then both guide wires 2 and 12 are pulled in order to retract the filters into sheath 10. Finally, both of the guide wires and sheath 10 are pulled as a unit out of the blood vessel. During any portion, or the entirety, of this step, suction may continue to be applied to filters 4 and 14 through sheath 10.

FIGS. 4A and 4B are simplified pictorial views showing two possible arrangements for a set of filters 4 and 14. The arrangement shown in FIG. 4A corresponds to that shown in FIGS. 1, 2 and 3. The arrangement shown in FIG. 4B differs in that filter 4 is inverted relative to the orientation shown in FIGS. 1, 2, 3 and 4A. The arrangement of filters shown in FIG. 4A is applicable to short, non tortuous segments of arteries. FIG. 4B shows an optional filter arrangement for longer segments of arteries especially if they are tortuous.

When the arrangement shown in FIG. 4B is employed, filters 4 and 14 are positioned in the blood vessel by the first and third steps as described above. In order to withdraw the filters, guide wire 2 is pulled to bring filter 4 into a position in which its large diameter end has been introduced into the large diameter end of filter 14. Then, as both filters are pulled into sheath 10, filter 14 is collapsed by its contact with sheath 10 and filter 4 is collapsed by its contact with the interior of filter 14. In this form of construction, filter 14 has an expanded diameter at least slightly greater than filter 4.

The arrangement illustrated in FIG. 4B offers the advantages that in the first step filter 4 can be extracted from sheath 1 somewhat more easily and, after filter 4 has been expanded, any debris produced by the operation performed in the second step will tend to collect near the apex of filter 4, away from its line of contact with the blood vessel wall.

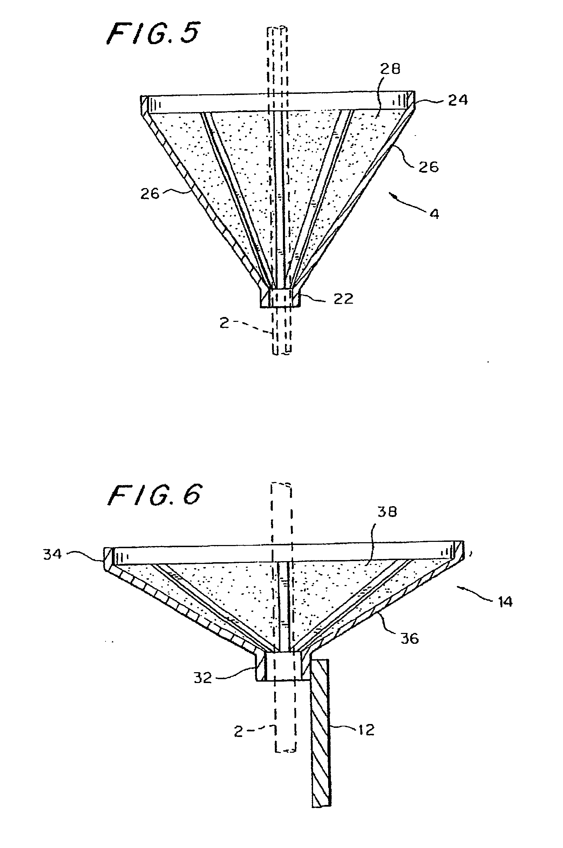

One exemplary embodiment of filter 4 is shown in greater detail in FIG. 5. This embodiment consist of a frame, or armature, composed of a small diameter ring 22 at the apex of filter 4, a large diameter ring 24 at the large diameter end of filter 4 and a plurality of struts 26 extending between rings 22 and 24. The frame is preferably made in one piece of a relatively thin memory metal, which is well known in the art. One example of such a metal is nitinol. The frame is constructed to normally assume a radially expanded state, such as shown at 4' in FIG. 1, but to be easily deformed so as to be retracted, or radially compressed, into sheath 1.

The frame is covered on its outer surface with a thin sheet, or membrane, 28 of suitable filter material having pores that are sized according to principles known in the art to protect organs downstream of the treatment site. The pore dimensions are selected to allow reasonable flow of blood to organs downstream of the treatment site when the filters are in place while trapping debris particles of a size capable of causing injury to such organs. The desired filtering action will be achieved with pore sized in the range of 50 .mu.m to 300 .mu.m. This allows different millipore sizes to be used to optimize either blood flow or embolism protection. The larger pore dimensions will be used in situations where a higher blood flow rate must be maintained and the escape of small debris particles is medically acceptable.

FIG. 6 is a view similar to that of FIG. 5 showing one suitable embodiment of filter 14, which is here shown essentially in its expanded state. Like filter 4, filter 14 includes a frame, or armature, having a small diameter ring 32 at its apex, a large diameter ring 34 at its large diameter end and a plurality of struts extending between rings 32 and 34. Filter 14 is completed by a filter sheet, or membrane, 38 secured to the outer surfaces of struts 36. Ring 32 provides a passage for guide wire 2, the passage being dimensioned to allow filter 14 to move freely along guide wire 2. Guide wire 12 is fixed to the outer surface of ring 32.

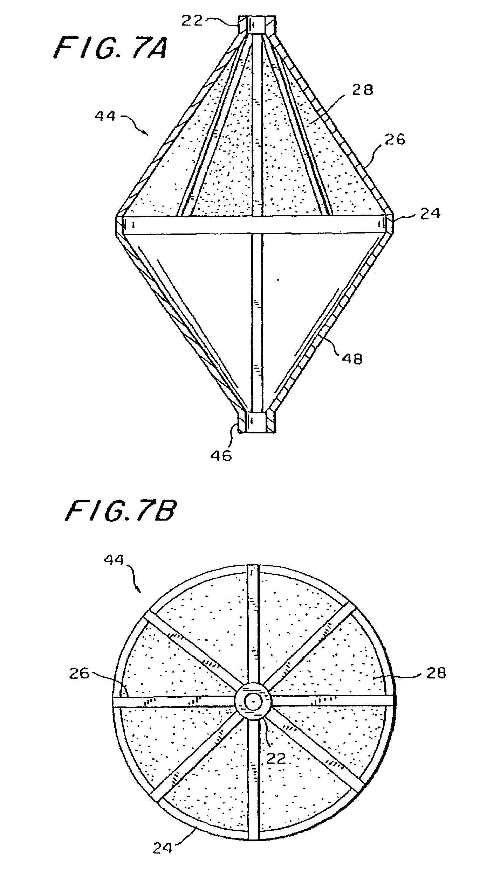

FIGS. 7A and 7B are, respectively, an elevational cross-sectional view and a plan view of another embodiment of a distal filter 44 that can be employed in place of filter 4. This embodiment includes, like filter 4, a small diameter ring 22, a large diameter ring 24 and a plurality of struts 26, with a filter sheet 28 secured to the outer surfaces of struts 26. Here again, ring 22 has an opening for receiving guide wire 2, which will be fixed to ring 22.

Filter 44 is further provided with a second, small diameter, ring 46 and a second series of struts 48 extending between rings 24 and 46. Ring 46 has an opening with a diameter larger then that of guide wire 2, so that ring 46 is moveable relative to guide wire 2.

All the parts of filter 44, except for membrane 28, like the corresponding parts of filter 4 and 14, may be made in one piece of a memory metal that has been processed to bias the filter toward its radially expanded configuration. All of these components are sufficiently thin to allow the filter to be easily collapsed radially within its respective sheath 1 or 10. Filter 44 will be mounted so that its apex faces in the distal direction, i.e. the cone formed by the struts 26 and filter sheet 28 have an orientation which is opposite to that of filter 4.

Filter 44 is brought to its radially expanded state in essentially the same manner as filter 4. When the filter portion is at the desired location in the blood vessel, sheath 1 will be retracted in order to allow filter 44 to expand radially. When the filters are to be withdrawn, guide wire 2 is pulled in the proximal direction until the lower part of filter 44, composed of ring 46 and strut 48, comes to nest either partially or fully in filter 14. Then, both guide wires 2 and 12 can be pulled in the proximal direction in order to retract the filters into sheath 10. During this operation, ring 46 has a certain freedom of movement relative to guide wire 2, which will help to facilitate the radial contraction of filter 44. Alternatively, or in addition, sheath 10 can be advanced in the distal direction to assist the retraction operation.

According to further alternatives, rings 22 and 46 can be dimensioned so that either guide wire 2 is fastened to ring 46 and movable longitudinally relative to ring 22, or guide wire 2 is fixed to both rings 22 and 46. In the latter case, radial contraction and expansion of filter 44 will still be possible in view of the flexibility and deformability of its components.

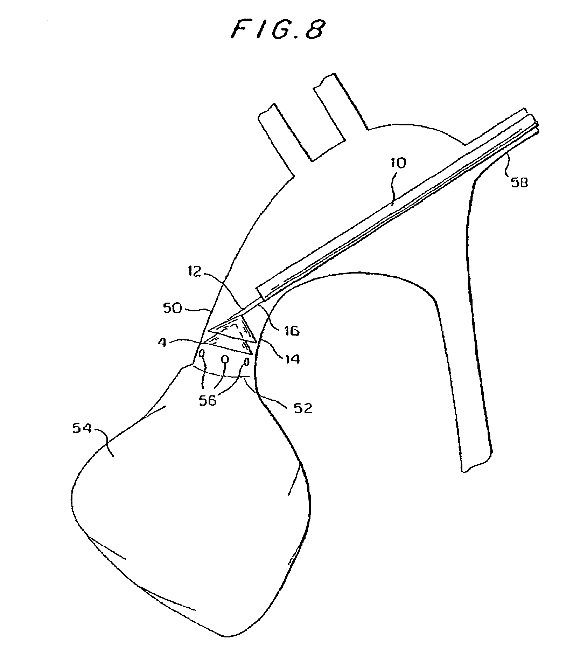

A system according to the invention can be used, for example, to improve the safety of bypass surgery. Referring to FIG. 8, an example of that surgery involves attaching vein bypass grafts to the aorta 50 starting from a point just downstream of the aortic valve 52 located between the left ventricle and aorta of the heart 54. In such a procedure, holes 56 are cut in aorta 50 for insertion of the upstream ends of the grafts. The operation of cutting into the watl of the aorta to sew on grafts can produce debris that will be carried along with blood flowing through the aorta to locations in the circulatory system where it can create an embolism in various organs, including the brain.

Referring to FIG. 8, the risk of such an occurrence can be reduced by introducing a system according to the embodiment of FIGS. 1-3, before holes 56 are cut, through a subclavian artery 58, which can be accessed via the patient's arm, and the brachial artery, to bring filters 4 and 14 to a location downstream of the location where holes 56 will be cut and to expand those filters so that they extend across the blood flow path through the aorta. Then, when holes 56 are cut, any debris produced by the cutting operation will be trapped, at least initially, within filter 4. However, while both filters are being withdrawn into tube 10, after holes 56 have been cut and possibly after vein grafts have been sutured to the holes, some debris may be squeezed out of filter 4, even as suction is being applied through tube 10. If this should occur, the debris can be drawn into filter 14 so as to be safely removed from the circulatory system.

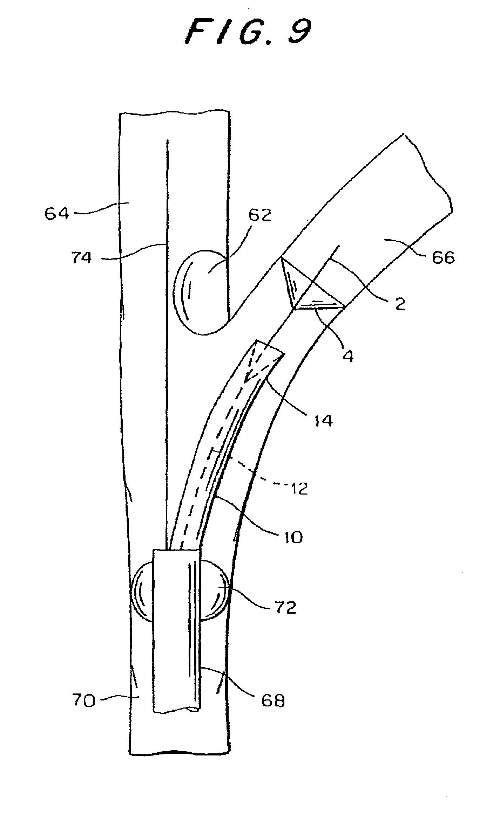

Another example of the use of a system according to the invention to capture debris incident to a medical procedure is illustrated in FIG. 9. A plaque deposit 62 is present on the wall of an internal carotid artery 64 just downstream of the junction with an associated external carotid artery 66. A guiding catheter 68 is introduced into common carotid artery 70 and is used as a conduit for introducing all other devices required to removes plaque 62 and collect the resulting debris. Catheter 68 carries an annular blocking balloon 72 on its outer surface and is provided with a conduit (not shown) for supplying inflation fluid to balloon 72.

A wire 74 carrying a Doppler flow sensor is introduced into internal artery 64 to position the flow sensor downstream of plaque 62. Then, sheath 1 (not shown) is introduced to deploy filter 4 in external artery 66, as described earlier herein and balloon 72 is inflated to block blood flow around catheter 68. After filter 4 is deployed and balloon 72 is inflated, any conventional procedure, such as described above with reference to FIG. 2, can be carried out to disintegrate plaque 62.

Then, as described with reference to FIG. 3, sheath 12 is advanced through catheter 68 to the location shown in FIG. 9, filter 14 is deployed and expanded into internal artery 66, and suction is applied as filters 4 and 14 are retracted into sheath 10.

In this procedure, starting from a time before disintegration of plaque 62, blood flow through common carotid artery 70 is blocked by inflated balloon 72. This results in a retrograde flow in internal artery 64 back toward common artery 70 and then antigrade flow into external artery 66, where debris being carried by the blood flow will be trapped on filter 4. The pressure sensing wire 74 is used to ascertain the collateral pressure, which must always exceed 40 mm Hg in the carotid. After a sufficient period of time has elapsed, filter 14 will be deployed to nest against filter 4 and both filters will be retracted into sheath 10 while suction is applied, possibly through sheath 10. Then, balloon 72 will be deflated, sheath 10 will be withdrawn through guide catheter 68 and catheter 68 will be withdrawn.

In another application of a device of the invention, the filters can be passed through a small peripheral artery into the aortic root to entrap debris generated during cardiac surgery. Such a device can be used during surgery or can be implanted for long-term use to prevent migration of blood clots to the brain under certain circumstances, such as during atrial fibrillation.

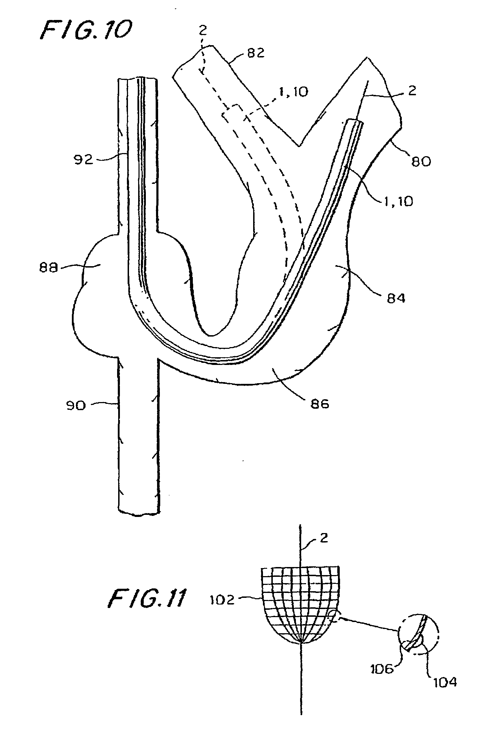

A further example of procedures that may be carried out with a device according to the invention is illustrated in FIG. 10, which shows the positioning of a device according the invention for treating an obstruction in an artery 80 or 82 emerging from the pulmonary artery 84 connected to the right ventricle 86 of a patient's heart. The right ventricle communicates with the right auricle 88 of the heart, which is supplied with blood from veins 90 and 92. In such a procedure, sheaths 1 and 10 may be introduced through either vein 90 or 92 and then through auricle 88, ventricle 86 and pulmonary artery 84 into either one of arteries 80 and 82 to be treated. Techniques for guiding the sheaths along the path illustrated are already well known in the art. Once positioned in the appropriate artery 80 or 82, an obstruction removal procedure will be performed in the manner described above.

FIG. 11 shows another embodiment of a filter component according to the invention in the general form of a basket, or cup, 102 made of a layer 104 of a radially compressible, autonomously expandable, material, such as a memory metal, and a filter sheet 106. Layer 104 may be fabricated by weaving memory metal wire into a mesh, or screen. Filter sheet 106 is made of a suitable plastic material, such as polyester, perforated to provide the desired filter pores, having dimensions described above. The bottom of basket 102 may be fixed to guide wire 2, in the manner of filter 4, described above, or may have a circular opening that is slidable along wire 2, with a second guide wire attached to the edge of the opening, in the manner of filter 14, as described above. Each such basket 102 will be used in the same manner as a respective one of filters 4 and 14 and will be dimensioned to extend across the blood vessel at the location where the system is to be employed.

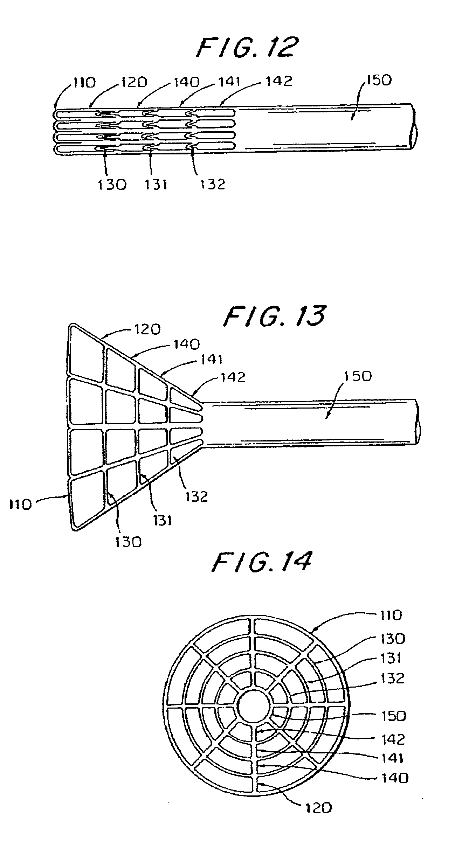

The procedures described above are merely exemplary of many procedures that can be aided by utilization of the system according to the present invention and other uses will be readily apparent to medical professionals. It should further be clear that the examples shown in the drawings are illustrated in a schematic form. For example the shape of the ring 24 in FIGS. 5, 7A and 7B is shown as a circle. However, for a ring that has to be collapsed to allow the filter to be pulled it into the sheath, it would be more logical to give it a slightly wavy or corrugated shape. This would make it more flexible and capable of smooth radial contraction and expansion. Another embodiment of a system having a distal protection system with a double filter according to the invention is shown in FIGS. 12-16.

In FIG. 12-14, a circularly cylindrical tube 150 is formed to have, at one end, which is here its distal end, a monolithic, or one-piece, distal filter that has a tubular conical shape with a pattern of slots that have been made in the surface of tube 150 by cutting, grinding, etching or any other technique. Tube 150 can be made of any material, like metal or polymer, and especially of nitinol with superelastic properties. Tube 150 may be long enough to be used as a guiding rail for catheters that are used for the angioplasty/stenting procedure.

At the distal end of tube 150, the slots are cut in such a way as to form a filter that has an expansion capability of at least, for example, a factor of 4. If tube 150 is made of nitinol, the expanded shape can be programmed into the memory by a heat treatment, while the material is kept in the desired expanded shape, shown in FIGS. 13 and 14, by some restraining tool. This is a known technique called shape setting.

The slots cut at the distal end of tube 150 leave thin, circularly curved, circumferential groups of distal strips 110 and groups of intermediate strips 130, 131 and 132. These strips are connected to, and interconnected by, thicker longitudinally and radially extending groups of struts 120, 140, 141 and 142 that end at the continuous, i.e., imperforate, surface of tube 150. Upon expansion for shape setting, struts 120, 140, 141 and 142 will bend out and give the distal section of tube 150 a conical shape. The thinner strips 110, 130, 131 and 132 will deform to follow circular arcuate paths during shape setting.

Tube 150 may have a length sufficient to have its proximal end (not shown) extend out of the patient's body where the surgeon can manipulate it. Tube 150 can also be shorter and attached to a separate guide wire to save costs or to reduce the diameter over the majority of the length.

The geometry of the strips and struts is chosen so that deformation upon shape setting and during expansion/contraction stays below acceptable limits. If necessary the cutting pattern of the strips can include some solid hinges. These are preferential bending spots, created by locally reduced thickness of the material. In this way it is also possible to cause a proper folding up of the strips while the filter is forced back into the cylindrical shape after conical shape setting.

In FIG. 12 the filter at the distal end of tube 150 is shown in its folded, or radially compressed, state, as it would appear when installed in sheath 1 of FIG. 1. FIGS. 13 and 14 show the final shape of the filter after shape setting and then after deployment from sheath 1. Distal strips 110 create a non-traumatic rim with a smooth series of tangential connections between the struts 120. The series of strips 130, 131 and 132 connect the long struts 120, 140, 141, and 142 together at different intermediate positions, but in principle intermediate strips 130, 131 and 132 could be omitted, at least if there are a sufficient number of longitudinal struts 120, 140-142 to create the desired fine mesh. However, the feasible number of struts is limited by the following parameters:

The initial tube diameter;

The minimum width of each slot, determined by the tooling;

The minimum required width for a stable strut; and

The desired expansion ratio determined by the acceptable length of each strut.

If the filter pores, constituted by the slots, are not fine enough, because the open area between the struts of an expanded filter becomes too large, additional circumferential groups of strips can be provided to make the mesh finer. The number of strips can be chosen freely, because they do not have an influence on the expansion ratio. For clarity only four rows of strips are shown in FIGS. 12-14. As can be seen, the length of the strips changes from proximal to distal. For example, strips 130 are longer than strips 131 and 132.

FIG. 14 shows a top view of the expanded filter where the strips 110 have been shape set to create a smooth rim that can perfectly cover the whole cross section of an artery with a good fit.

The conical filter shown in FIGS. 12-14 is meant to be used in combination with a delivery sheath, as described herein with reference to FIG. 1. Such a sheath can run over the surface of tube 150 and if the sheath is retracted, the filter will assume the conical shape shown in FIGS. 13 and 14, which is substantially the same as the shaping pattern of FIG. 1. When such a delivery sheath, surrounding a collapsed filter, is brought into an artery and then gently withdrawn, the filter will open up, flare out and completely obstruct the cross section of the artery. Nitinol is an excellent material for such a filter, because it can withstand high elastic strains. A nitinol filter according to this design can be deployed and collapsed elastically several times without any plastic deformation, whereas known filter materials would fail.

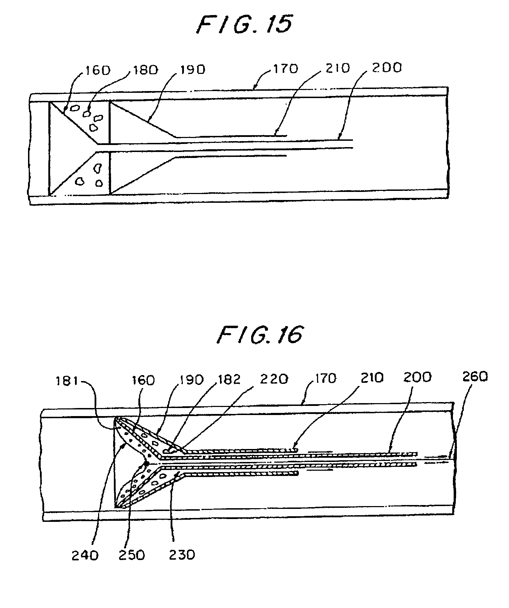

In FIG. 15 a pair of filters 160 and 190 each having the form shown in FIGS. 12-14 according to the invention are used in combination in order to entrap emboli particles between them for removal from the artery.

During the major part of an angioplasty/stenting procedure, only the most distal filter 160 is in place. During angioplasty/stenting of the artery 170, emboli particles 180 may be released from the lesion site and move with the blood stream until they are stopped by filter 160. At the end of the procedure, a second filter 190 is advanced over the wire or tube 200 that is connected to filter 160. The diameters of the distal ends of filters 160 and 190 are about the same, and filter 190 can completely be advanced over filter 160, when it is delivered from its own delivery sheath (not shown). Filter 190 has its own tube 210, which has a much larger inner diameter than the outer diameter of wire or tube 200 of the first filter 160. The lumen between both tubes 200 and 210 can be used for flushing/suction. Of course this can also be performed through tube 200 as well.

FIG. 16 shows the system of FIG. 15, with the thickness dimensions of the various components illustrated more clearly, at a point in a procedure just after the second filter 190 has been brought into a position to enclose the first filter 160, with the distal ends of both filters in contact with one another. The opening angles of both filters may be identical or, as shown, different. In case they are identical, the surfaces of both filters will mate perfectly and all debris will be trapped, like in a sandwich, between the two conical surfaces.

However, if the cone of the second filter 190 has a smaller opening angle than filter 160, as shown, the situation shown in FIG. 16 will result. The distal edges of both filters fit well together, but for the rest there is a gap between the surfaces of the two filters. This gap creates a chamber 220, in which small particles can freely move. The advantage of this arrangement is that the particles can be removed from chamber 220 by suction through the lumen 230 between tubes 200 and 210.

FIG. 16 further shows an additional filter sheet 240 that is used to capture fine particles that go through the holes in filter 160. The holes in the filter 160 can for example have a maximum size of 250 .mu.m, while filter sheet 240 can be provided with holes, or pores, having a size of the order of only 150 .mu.m or less, dependant on the application.

Filter sheet 240 may be made of a fine metal sheet, a polymer, or any other flexible tissue and it can be attached to the distal strips 110 of filter 160 by means of glue, stitching or any other means. At its proximal extremity, corresponding to its center, sheet 240 may a central connection point 250 that is connected to a long wire 260 that runs completely through tube 200 to a location outside of the patient's body. With this wire 260, filter sheet 240 can be pulled into a conical configuration before filter 160 is pulled into its delivery sheath (not shown). This makes it easier to bring filter 160 and filter 240 into a smooth collapsed state. Once filter 160 is deployed, or expanded, wire 260 may be released a little bit to enable filter sheet 240 to move away from filter 160, thus creating additional space for entrapment of the small particles 181 that fit through the holes in filter 160. The larger particles 182 will not go through filter 160 and will stay at the proximal side of this filter. If chamber 220 between the conical surfaces of filters 160 and 190 is large enough, and if wire 260 of filter sheet 240 is not pulled too tight, most particles can easily be suctioned out through lumen 230. By pulling wire 260, the particles 181 will be forced to move in the direction of the suction opening. This is another advantage of the use of a movable filter sheet 240.

Finally only some very large particles will remain in chamber 220, and they can be removed by holding them entrapped between the surfaces of the filters, while both filters are pulled back into the delivery sheath and the filters are compressed, or collapsed to their cylindrical configurations. This is done while continuous suction is applied.

In case the large particles are squeezed, break up and slide through the holes in filter 160, they will again be gathered in filter sheet 240. Eventually wire 260 can be released even more if there is a lot of material between filter 160 and filter sheet 240. In that case, filter sheet 240 may look like a bag, filled with material, that hangs on the distal side of the completely collapsed filter 160. This bag may not be pulled back into the delivery sheath, but will just be pulled out of the artery while it hangs at the distal tip of the sheath.

A major advantage of this double filter design is that upon compression of the filter cones, the emboli particles can only leave the chamber 220 through the suction lumen 230, or they stay there to be finally entrapped mechanically between the cone surfaces or to remain in the bag.

The distal filter will be in place during the whole procedure of angioplasty/stenting and therefore the mesh size is very important. An additional pressure-measuring tip, distally in the blood stream may monitor perfusion. The wire that holds this tip may be integrated with wire 260 that is controlling the filter sheet 240. Alternatively, wire 260 can have the form of guide wire 2 shown in FIG. 1, with a lumen connected to a pressure detector.

On the other hand, filter 190 is only used a very short time and therefore its mesh size may even be finer than that of filter 160.

As explained above, the number of longitudinal struts is limited on the basis of the desired expansion ratio. The distance between two circumferential strips can be made rather small, but they must still be able to be bent in order to get a collapsable and expandable device. Therefore a certain gap must remain between them. Normally such a gap would be larger that 50 .mu.m, so an additional filter mesh is required in case the allowed particle size is 50 .mu.m, such as for use as a filter in a carotid artery.

In general, filter systems according to the invention can have many embodiments, including systems containing a distal filter with or without an additional filter mesh with a proximal filter, also with or without an additional filter sheet. Also the relative position of filter and filter sheet can be varied. The sheet can be outside of filter 160. Further embodiments can be combinations of emboli catching devices of different geometries and/or types. Filters, balloons and sponges of all kinds can be used in multiple combinations, all based upon the principle of full entrapment of particles before the protection device is collapsed upon removal from the patient's body. Combinations of an inflatable delivery sheath according to the invention with a multi-filter arrangement, as disclosed, are also meant to be an embodiment of this invention.

FIGS. 17-27 illustrate the structure and successive phases in the use of another embodiment of the invention that is suitable for performing angioplasty procedures while trapping and removing debris produced by the procedures.

FIG. 17 shows an artery 302 with an obstruction, or lesion site, 304 that reduces the effective diameter of artery 302. The invention can be used to treat virtually any artery throughout the body, such as for example the inner carotid artery where emboli are extremely dangerous because the particles can cause stroke in the brain.

A first component of this embodiment is a guide wire 306 that, in a first step of a procedure using this embodiment, is advanced through artery 302, normally in the direction of blood flow, and past lesion site 304. The blood pressure in artery 302 adjacent the distal end of guide wire 306 can be monitored by a pressure monitoring device that includes a miniature pressure sensor, or transducer, 310 at the distal end of guide wire 306 and a signal measuring unit at the proximal end, as represented by element 5 in FIG. 1. Guide wire 306 can be provided with a longitudinal lumen that can contain wires or an optical fiber to transmit electrical or optical signals from sensor 310 to the signal measuring unit and the signal measuring unit can be connected to a conventional indicator, display and/or warning device. Sensor 310 may be, for example, a distal miniature load cell, possibly of the type having a load-dependent electrical resistance. The pressure monitoring device can continuously monitor the blood pressure in artery 302 during an entire procedure.

FIG. 18 shows the second step in which a guiding catheter, or sheath, 312 having a longitudinal lumen carrying a distal protection means 314 is advanced over guide wire 306 until means 314 reaches a location that is distal, or downstream, of lesion site 304. If distal protection means 314 is a filter made from a small slotted nitinol tube, it can be advanced over guide wire 306 while being retained in the lumen that extends through catheter 312.

Distal protection means 314 may be a filter, as described earlier herein. For example, means 314 may be an expandable filter cone, or umbrella, having the form disclosed, and deployed and retracted in the manner disclosed, earlier herein with reference to FIGS. 1-14, and particularly FIGS. 12-14, held in its collapsed state within catheter 312.

In the next step, depicted in FIG. 19, the distal protection means 314 is deployed until it extends completely across the blood flow path defined by artery 302 in order to catch all emboli particles that may be released from the lesion site upon the following steps of the procedure. Protection means 314 will stay in place until the end of the procedure.

FIG. 20 shows the following step in which a predilatation catheter 320 is introduced over guiding catheter 312. Predilatation catheter 320 carries, at its distal end, a predilatation balloon 322. Predilatation catheter 320 can be advanced over guiding catheter 312 and has several purposes. First, its predilatation balloon 322 can be used to enlarge the inner diameter of lesion 304 in order to create sufficient space for positioning a post-dilatation device 326 in the form of a sheath carrying an inflatable balloon section 328. Section 328 may, if desired, carry a stent 332 that is initially in a radially contracted, or collapsed, state. Furthermore the distal tip of the catheter 320 with balloon 322 can act as an internal support for the post-dilatation balloon 328. The inner wall of device 326 constitutes a delivery sheath within which self-expanding stent 332 is retained prior to deployment and out of which stent 332 can by pushed by some conventional delivery means (not shown). Such a delivery means for self-expanding stents can be of any kind, for example a pusher-wire that pushes against the proximal side of the stent to push it out of the sheath.

FIG. 21 shows the subsequent step in which predilatation balloon 322 has been deflated and advanced in the distal, or downstream, direction. Self-expanding stent 332 has been pushed out of delivery sheath 326. Normally, a delivery sheath only serves to bring a stent in its compressed state to the lesion site and to hold it compressed until it is to be deployed. This sheath generally has a cylindrical shape and upon delivery of the stent the sheath is pulled back, while the self-expanding stent leaves the distal tip of the delivery sheath. The sheath is then removed from the patient's body. The stent may have enough radial expansion force to fully open at the lesion site, but often this force is insufficient and the stent will stay in some intermediate semi-deployed position. A self-expanding stent can be made of several types of material, for example nitinol. Nitinol is a material with mechanical hysteresis and the force needed to collapse the stent is much higher than the radial force that the stent exerts upon deployment. This means that a nitinol self-expanding stent may be strong enough to hold an artery open, but it may need some help to reach full deployment. This help can come from post-dilatation balloon 328.

FIG. 22 shows the next step in which sheath 326 is used to help deploy stent 332. The distal end of sheath 326 with balloon section 328 can be inflated through a lumen (not shown) in the sheath wall. First the delivery sheath 326 is advanced again and the balloon area 328 is lined up with stent 332 in lesion site 304. Inflation of balloon section 328 will now cause further expansion of stent 332. However, the inner wall of sheath 326 that held stent 332 before delivery may collapse under the high pressure that may be needed to fully deploy stent 332. Therefore, predilatation balloon 322 can be inflated to be used to create a stiffer inner support for sheath 326. By lining up of both balloon sections, as shown in FIG. 23, a concentric double balloon segment is created, which is strong enough for post-dilatation.

FIG. 24 show the next step in which stent 332 is fully deployed by the combined forces of balloon 322 and post-dilatation balloon section 328, despite the opposing forces of the artery wall at lesion site 304 that now has become a larger opening.

FIGS. 25 and 26 show the next step in which predilatation catheter 320 has been removed, leaving inflated balloon section 328 around delivery sheath 326 in place. Although the internal support for sheath 326 has been removed, inflated balloon section 328 can easily be used for proximal occlusion means, because the pressure may be much lower than for post-dilatation of the lesion and stent deployment. Sheath 326 that held stent 332 before can now be used as a working channel, e.g. for flushing and suction. This working channel is in open connection with devices outside of the patient's body and can be used for a series of procedures in the closed chamber 336.

If desired, the inflatable delivery sheath/suction tube 326 can be deflated, pulled back until it is proximal of the stent section and then be re-inflated to enable additional flushing, suction and inspection, while the distal occlusion device 314 is still in place.

For supply of flushing fluid, a separate lumen can be made in the wall of delivery sheath 326, running to the distal end of this sheath (not shown). Other procedures in a temporary closed chamber of an artery include ultrasonic treatment, radiation therapy and drugs delivery, among others.

FIG. 27 shows a final step in which post-dilatation balloon section 328 has been deflated and distal protection means 314 has been collapsed. The final step can be the removal of all devices from the patient's body, except, of course, stent 332, which can stay there.

FIGS. 28-40c show the present invention embodied as filters that can serve as distal or proximal filters in the two-filter systems shown in FIGS. 1-27, where FIGS. 28-31 particularly show a manufacturing technique that can also be used in the manufacture of the filters, as well as non-filter devices. By the present invention, filters with improved flexibility and smaller profile are described. Such a filter includes a proximal frame for expansion and contraction and a thin filter bag attached to the frame. The filter is a composite of two basic materials. In the present context, a "composite" structure is distinguished from other reinforced devices where discrete structural members are connected to or supportive of relative non-structural members without substantial integration of the two. By contrast, a composite structure includes at least a relatively non-loadbearing matrix member that surrounds (or embeds) a loadbearing reinforcement member such that the two are integrally formed to define a unitary member. With this understanding, a substantially monolithic membrane (made form, for example, a polymeric or related plastics material) merely attached to an underlying or overlying structural cage or basket (made from, for example, a metal or plastic material) is more akin to a "body-on-frame" structure rather than a "composite" structure. In the present invention, the first (matrix) material makes up the highly flexible filter membrane, where a pattern of holes in the membrane allows the flow of blood particles below a well defined size. The second (reinforcement) material is one or more fibers that possess high axial strength, but are thin enough to be flexible upon bending. The reinforcement is integrated with the membrane to create a composite structure with very flexible membrane areas where the blood is filtered, and extremely strong reinforcement fibers that take up excessive forces. The strength of the fibers prevents the membrane from tearing even in response to pulling or related moving forces, while their flexibility allows hinging at the points of attachment to the proximal frame and/or to an elongated member used for transporting the membrane to or from the location within the patient's body where the membrane is needed. The elongated member can be one of numerous conventional devices, including (but not limited to) a guide wire, a hollow tube, a tool for holding the aforementioned proximal frame, or a balloonable stent.

All of the fibers disclosed herein can be made from a variety of materials, including (but not limited to) Dyneema®, an extremely strong polyethylene manufactured by DSM High Performance Fibers, a subsidiary of DSM N.V. The fibers can also be combined with fibers or wires of other materials, such as Nitinol (a version of shape memory nickel-titanium alloy), to help control the expanded shape of the filter. Other viable materials for use as reinforcement fibers include those known in the fiber art, such as carbon, glass, ceramic, metals and metal alloys (including the aforementioned Nitinol), polymers (including ultra high molecular weight highly oriented polymers) or combinations thereof Moreover, the reinforcement fibers can be made of a monofilament or multi-filament, and can be configured to have all kinds of cross sections and orientations. The fibers can be made of round, flat or different shaped monofilaments or multi-filaments. Preferably, the material making up the fibers has a modulus of elasticity that is higher than that of the surrounding membrane.

As part of a composite structure, the reinforcement fibers are integrated (embedded) into the membrane. The fibers can also be attached to the frame by any known technique, including the use of dipping, spraying, welding, glue, stitching, sewing, pressing, heat, light and knotting. Moreover, the fibers can be distributed over the membrane surface in a specific designed pattern or in a random pattern. In addition, the reinforcement fibers can be either continuous or discontinuous. With continuous reinforcement, the fibers are made up of one or more long strands that span the substantial entirety of the component they are reinforcing, forming a substantially rigid backbone-like structure. With discontinuous reinforcement, the fibers are shorter, typically made of numerous chopped, discrete strands that are interspersed throughout the component they are reinforcing. Even relatively short pieces of discontinuous fiber embedded into a membrane can reinforce such a membrane considerably. This is caused by the relatively short distance between adjacent fiber pieces, thus enabling distribution of applied forces to neighbor fibers. Forces can be taken up by fibers with different orientations and such fibers can either be embedded in a specific pattern or randomly distributed pattern. Combinations of long fibers and short fibers are also possible. The long fibers can for example be used for attachment to the frame and the short fibers may be used to improve the characteristics of the membrane itself Continuous reinforcement generally provides higher loadbearing capabilities and crack formation and propagation resistance, while discontinuous reinforcement generally facilitates lower cost and more complex finished composite structures. As such, the orientation and number of the reinforcement fibers is not limited and can vary with the desired application. In order to achieve a better connection between the reinforcement fibers and the membrane material, the fibers may first be coated with a material that adheres well to the membrane material, for example with the same material as the membrane.

The reinforcement fibers not only improve the strength of a membrane, but also can prevent stress degradation and improve the fatigue properties of heavily-loaded membranes (such as those employed in a heart valve). In addition, pulling fibers can also be used for enabling the removal of a medical device by pulling the device into a removal sheath, as will be discussed in more detail below. In this latter configuration, the pulling fibers may be embodied by either a single pulling fiber or multiple fibers. In addition, the pulling fibers can be made from the same material as the reinforcement fibers. In either case, the fiber(s) may be actuated directly by the operator, or indirectly by the guide wire through a stop as described below and in conjunction with the filter design. In addition, the fibers can be used to control the final geometry, prevent crack propagation, act as hinges at the place of attachment to the frame and prevent loss of the membrane or parts of it. Because the reinforcement enables the membrane to be made much thinner than known membranes, the crossing profile of the composite filter can be much lower than for a single polymer membrane, even if the reinforcement fibers are thicker than the membrane itself.

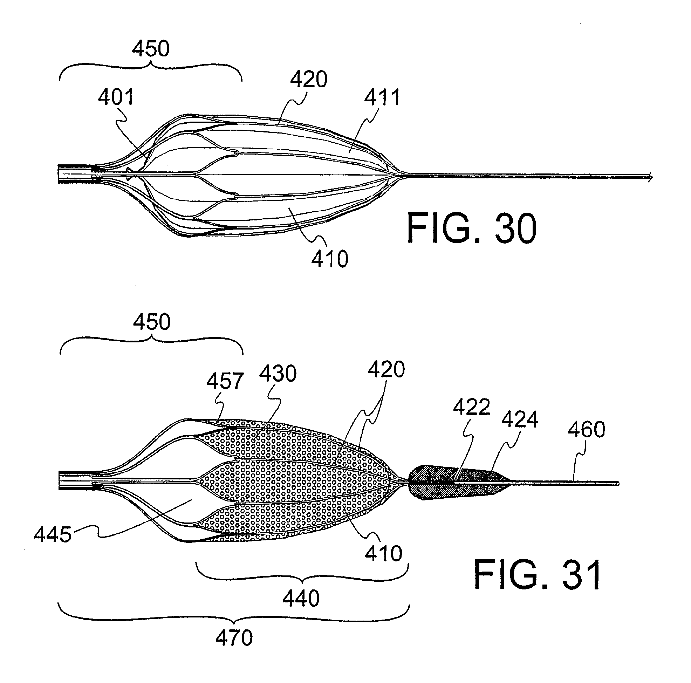

Referring next to FIGS. 28 through 31, a method for making a reinforced filter is carried out by first providing a paraffin mold 401 having the desired shape of the expanded, or deployed, filter bag. Paraffin is chosen because it can be removed from the filter easily at a temperature that does not cause degradation of the finished filter. In addition, with the use of a paraffin mold 401, it is possible to make complicated or simple designs, because there is no need to remove a relatively large mandrel from the finished product after it has been made. Paraffin is of course not the only material that can be used for mold 401; any material that can be brought into the desired shape and can be dipped directly or after application to an intermediate layer may be used. Examples are meltable materials or materials that easily dissolve in water, including salt or sugar crystals. Other examples are fine grains in a vacuum bag or an inflated balloon which is deflated after dipping. It is also possible, for certain filter embodiments, to use a mold that can be safely removed without being melted, dissolved, or deformed. To improve the quality of the dipping process between paraffin and certain polymer (such as polyurethane), the paraffin mold 401 is first covered with a thin sheet 402 of polyvinyl alcohol (PVA). The PVA 402 is a thin sheet that can be stretched after wetting with water and pulled tight around the mold 401 and then tied together with a small clip or wire 403. The resulting mold 401 is dipped a few times in a solution of polyurethane in tetrahydrofuran, thus building a skin (or layer) of polyurethane. By way of example, this skin can be approximately 3 microns thick. After this, mold 401 (covered with the polymer skin) is dipped in a solution of polymer and solvent until a membrane 410 is created. Referring next to FIG. 29, a frame 450 is then placed around the mold 401, and reinforcement fibers 420 (which may be coated) are then mounted to the frame 450 at hinge sites 459 and laid over the surface of the mold 401. While discontinuous fibers can be used to improve the structural properties of membrane 410, it will be appreciated by those skilled in the art that the connection between membrane and the frame is enhanced when the hinge sites on the frame can be tied to reinforcement fibers in the composite structure. As such, a more secure connection is possible with continuous reinforcement fibers than with discontinuous fibers, as the continuous fibers can be looped around or otherwise tied to the frame's hinge sites. Additional dipping into the solution of polymer and solvent ensures full embedding of the fibers 420 into the growing polymer layer membrane 410 shown in FIG. 30. Finally, a perfusion hole pattern made up of holes 430 is laser drilled into the membrane 410, as shown in FIG. 31. The size of the holes 430 are such that blood or related fluids can pass through, while inhibiting the passage of solid objects (such as a disdlodged emboli). Preferably, the size of the holes 430 is up approximately 100 microns in diameter, although it will be appreciated that other sizes, depending on the application, can also be employed. While the holes 430 in membrane 410 are distributed over the membrane surface in a specific designed pattern, it will be appreciated by those skilled in the art that the holes 430 could also be disposed in a random pattern, even if they cut through the reinforcement fibers 420. After drilling of the holes 430, the central paraffin mold 401 is removed by melting in warm water, which can be at a temperature of 50° C. The PVA 402 is easily released from the polyurethane membrane 410 and is removed. Once the membrane 410 is created, the polymer skin can be easily detached from the inside of the membrane 410 and pulled out. The surface of the membrane 410 may additionally be coated with another material, such as biocompatibility-enhancing materials or drug-release agents. While much of the discussion herein relates to a polymer-based membrane 410, it will be appreciated that other materials could also be used to form membrane 410, including organic tissue and tissue from human or animal origin, although the fabrication methods may be different than that depicted in FIGS. 28-31.

Referring again to FIG. 29, frame 450 is made of Nitinol (or similar shape memory alloy) tubing having an outer diameter of 0.8 mm by laser cutting and shape setting. At the proximal (left-hand) side, tube 455 is in its uncut state, and still 0.8 mm. in diameter. From there, tube 455 is cut to form eight longitudinal spokes 456 that end in a zigzag section with struts 457, where the unconstrained, expanded material of frame 450 lies on a circle having an 8 mm diameter at its largest point. This frame 450 will, at any size between the maximum diameter and the collapsed size of 0.8 mm diameter, always adapt smoothly to the given geometry of the body lumen, such as an artery. Eight reinforcement fibers 420 are attached to the most distal section of frame 450 at hinge sites 429. Fibers 420 can be attached to frame 450 by means of a knot or each fiber 420 can just be run back and forth from a distal location to hinge sites 429 and wrapped around frame 450 at that location. In the latter case, each fiber 420 will have twice the length shown. At the distal (right-hand) end of frame/filter assembly 470, all fibers 420 converge in a guide tube 405, where they are held in correct position for additional dipping operations.

Referring again to FIGS. 30 and 31, mold 401, frame 450 and the surrounding fibers 420 are shown after having been dipped enough times to embed the fibers 420 into membrane 410. By way of example, membrane 410 is 5 microns thick at places 411 where no reinforcement fibers 420 are present. Guide tube 405, mold 401 and PVA 402 are removed after the dipping is finished and the membrane 410 has dried, as previously mentioned. FIG. 31 shows the final filter 440, with a pattern of laser drilled holes 430 between the reinforcement fibers 420. Further, the fibers 420 are cut to the correct length at point 422 and attached to a central guide wire 460 via connector in the form of a nose tip 424. The nose tip 424 can fit on top of a delivery catheter if the filter 440 is retracted into the catheter before placement into the body lumen of the patient. Note that the membrane 410 between the struts 457 at the distal end of frame 450 and the dipping line is removed, preferably by laser cutting. Filter mouth 445 is where the proximal end of filter 440 meets the distal end of frame 450. The construction of the frame/filter assembly 470 is extremely strong and still very flexible. The 5 micron thick membrane 410 with the reinforcement fibers 420 fits easily in a delivery catheter of only 0.9 mm inner diameter and adapts to all sizes of arteries between 1 and 8 mm diameter.

The central guide wire 460 extends to the left from connector 424 through the membrane 410 and frame 450, including the uncut part of tube 455. Within connector 424, fibers 420 are wrapped around, and secured to, guide wire 460. To remove the filter 440 from a delivery catheter, guide wire 460 is pushed from its proximal (left-hand) end (not shown) so that a pulling force is exerted on fibers 420 due to their connection to guide wire 460 in connector 424. Thus, all tension forces on the distal section of the filter 440 are taken up by the reinforcement fibers 420. The membrane 410 only has to follow these fibers 420 and unfold as soon as it leaves the catheter. The filter 440 opens because of the elasticity inherent in frame 450. In addition, the blood pressure in the artery further helps to open the filter 440 like a parachute. Upon bending of the filter 440, there is almost no force needed at the hinge sites 459 where fibers 420 are attached to the struts 457, so these sites (as their name implies) act as hinges. Even in highly curved arteries, the filter 440 and frame 450 still adapt well to the artery wall, resulting in almost no blood leakage between the membrane 410 and artery wall.

The fibers 420 are so well embedded in the membrane 410 that even if the membrane 410 were to detach from a strut 457, the membrane 410 will still have a strong connection to the frame 450 and can be collapsed and removed from the patient safely. In case of a tear in the membrane 410, for example starting from one of the holes 430, the presence of the fibers 420 bridges the crack, thus stopping the tear. This crack-bridging occurs with both the shown continuous fibers, as well as with discontinuous fibers (not shown), as previously discussed. While any breach in membrane 410 is capable of liberating previously captured emboli to a downstream position in a body lumen, the composite nature of the present device not only keeps the size of the breach to a minimum (thereby minimizing such emboli liberation), but also reduces the likelihood of pieces of filter 440 breaking off and passing through the lumen.

After a medical procedure has been performed, the frame 450 can be collapsed to close the mouth 445 of filter 440, and entrapping emboli and related debris therein, as the filter 440 takes on a bag-like appearance. The hinged nature of the filter/frame interface guarantees that the filled bag hangs at the distal end of the removal catheter and still can move easily through curved arteries.

As previously mentioned, the reinforcement fibers 420 can be used not only for their high tensile strength, but also can be combined with memory metal wires, or filaments. These can be, for example, Nitinol wires that can be shape set to almost any desired shape by heat treatment. Such wires may be embedded in or attached to the membrane 410 to guarantee a smooth folding/unfolding of the membrane 410. An example is an embedded Nitinol wire that helps to give the mouth 445 of the filter 440 a smooth geometry that fits well to the artery wall. Such a Nitinol wire for shape control can be combined with a more flexible, but stronger, fiber, which is used to protect the membrane 410 of filter 440 against incidental overload, tear propagation or related problems that plagues non-reinforced membranes.

Referring next to FIG. 32, an alternate embodiment of the medical device of FIG. 31 is shown, where a filter 540 is formed from a conical shaped membrane 510. As with the embodiment depicted in FIGS. 29-31, the filter 540 is attached to frame 550, although in the present case, the membrane 510 is not attached directly thereto. Instead, it is attached by a single reinforcement fiber 520 from the distal end of guide wire 560 until it reaches the struts 557 at hinge sites 559, at which point it then wraps back to the distal tip of guide wire 560 with a reverse angle. Arrows in the drawing show how fiber 520 runs back and forth. By this method the use of knots at the fiber/frame interface is redundant and the safety is further increased, because the filter 540 can never detach from the frame 550. As with the previous embodiment, membrane 510 can also be formed by dipping a suitably shaped mold (not shown) in a solution of polymer and solvent. Guide wire 560 is fastened to fiber 520 at least one point at the distal end of the filter 540 and extends therethrough to a proximal (left-hand) end thereof. The pattern of crossing reinforcement fibers 520 gives the filter 540 different elastic properties, including improved axial elasticity. The pattern of holes 530, preferably cut by laser, can be made in zones between the fibers 520 to avoid damage thereto. However, if the pattern of reinforcement fibers 520 is very fine, the holes 530 may be placed without regard to fiber 520 location, as there will still be enough reinforcement left even if some of fibers 520 are cut. The presence of adjacent crossings and parallel or angled uncut fibers 520 can take over some of the load-carrying capability, as can the embedding material of the membrane 510. The conical shape of filter 540 is advantageous in that if it has a maximum expanded diameter of 8 mm, and is placed in an artery of 8 mm diameter, all holes 530 will be free from the artery wall and blood can flow through all holes 530. As soon as debris, such as dislodged emboli, are entrapped, they will tend to collect at the most distal tip, leaving the more proximal holes open.

The area of the conical surface of filter 540 relates to the cross-sectional area of the artery as the length of the cone edge from base to tip relates to the radius of the artery. Preferably, the total surface area of the holes 530 should be at least equal to the cross-sectional area of the artery in order to guarantee an almost undistorted blood flow. This is the case if the ratio of the total surface area of the cone surface to the total hole surface area is smaller than the ratio of the cone surface area to the cross-sectional area of the artery, or, in other words, the total surface area of the holes 530 is at least equal to the cross-sectional area of the artery. For an artery having an inner diameter of 8 mm, a total number of 6400 holes 530 each with a 100 micron diameter is needed for the same surface area. While the type of flow through numerous small diameter holes is different from the undistorted flow through an open 8 mm artery, because the wall thickness of a reinforced membrane according to the invention can be extremely small, the length of a hole (for example only 5 microns, the thickness of the membrane) ensures a much better flow than a comparable-diameter hole in a thick membrane. The use of reinforcement fibers 520 makes it possible to reduce the thickness of membrane 510, such that the flow resistance through the membrane wall decreases, allowing filter 540 to act as a semi-permeable membrane. A filter 540 made in conical shape will also have enough free holes 530 if used in arteries with smaller diameter. The holes 530 that touch the artery wall will not contribute to the flow, but the remaining holes 530 not in contact will have the same surface area as the actual cross section of the smaller artery.

Filters according to this invention are more flexible than existing filters so that they can be made longer without creating problems in highly curved body lumen. This increase in length promotes greater storage capacity for dislodged emboli. If the reinforced membrane 510 and frame 550 are mounted to each other without overlap, as in FIG. 32, the collapsed diameter can be made even smaller than with the embodiment shown in FIG. 31. Here, at a specific cross section of frame 550 near the hinge sites 559, the frame 550, membrane 510, fibers 520 and central guide wire 560 cooperate to fit within the available cross section in the delivery sheath. The present construction of frame 550 has certain advantages. For example, production of frame 550 is very simple, guide wire 560 is kept in the center, and the filter 540 can be pulled out of the delivery sheath by pushing on guide wire 560 from the left to exert a pulling force on fiber 520 and membrane 510.

During removal of the filter 540 from an artery, the longitudinal spokes 556 of frame 550 just have to pull the struts 557 of the zigzag section into a removal sheath. However, there may be circumstances (such as highly curved body lumen) where it is desirable to avoid having the guide wire 560 bend to the point where it interferes with or deforms the zigzag struts 557. Similarly, there may be procedures (such as angioplasty/stenting) where axial movements of the guide wire 560 caused by the procedure can influence the position of the filter 540. It would be better if the guide wire 560 could move freely over at least a certain axial length, as well as in radial and tangential directions, within the entire cross section of the filter 540, without exerting any force on the expanded frame 550.

Referring next to FIGS. 33-36, another alternate embodiment of the present invention with such a freely movable guide wire 660 is disclosed. FIG. 33 shows a filter 640 in an expanded state such that it and frame 650 occupy a large profile. Filter 640 is constructed in such a way that it can be conveyed from a delivery sheath by pushing on guide wire 660 to exert a pulling force on filter 640. After completion of use of the filter 640 in a medical procedure, it is removed by pulling it into a removal sheath with the aid of guide wire 660. The pulling forces are applied in both directions by moving guide wire 660 in axial direction relative to the sheath. Guide wire 660 runs through the filter 640 and ends at guide wire distal section 662. Fastened to guide wire 660 are stops 663 and 664 that have a larger diameter than the guide wire itself. These stops are connected tightly to the guide wire 660 by any known technique. At the distal tip of filter 640, a ring 665 is fastened to the filter, while guide wire 660 can slide freely through ring 665 until stop 663 touches ring 665. At the proximal side of stop 664, a second ring 666 is mounted around guide wire 660 to allow it to slide freely therethrough. As such, both rings 665 and 666 are slide rings, and are given a smooth shape with rounded leading edges to let the guide wire 660 move easily in associated sheaths and in the artery. As can be seen in the figures, the slide rings 665, 666 can be connected to the filter 640 by reinforcement fibers 620, pulling fibers 625, membrane 610 or combinations of the above. It will be appreciated by those skilled in the art that the pulling fibers 625 may be made from the same or differnet amterial as the reinforcement fibers 620, depending on the need. In the embodiment shown, pulling fibers 625 are generally configured to carry the pulling load in the proximal (leftward) direction, while reinforcement fibers 620 are generally configured to carry the pulling load in the distal (rightward) direction. In the strictest sense, while reinforcement fibers 620 also perform a pulling function (at least in the distal direction associated with insertion of the device into an appropriate body lumen), their nomenclature in this disclosure is retained to make it clear that they alone can perform the dual function of reinforcing the composite structure as well as bear a pulling load. As such, the distinction between the purely pulling capacity of pulling fibers 625 and the aforementioned dual function of reinforcement fibers 620 should be apparent from the context. Membrane 610 is connected directly to slide ring 665, as are reinforcement fibers 620. At the other side, reinforcement fibers 620 are connected to expandable frame 650 at hinge sites 659, possibly together with the material of membrane 610. Expandable frame 650 is provided with attachment points 658 at its proximal side, which are needed to pull the frame 650 back into a removal sheath 600, shown in FIG. 34. Pulling fibers 625 (which, as previously discussed, may be made from the same or different material as reinforement fibers 620) are connected to the attachment points 658 of the proximal section of frame 650 and run to the proximal slide ring 666, to which they are securely attached.

If the guide wire 660 is moved through the filter 640 in the proximal (leftward) direction, stop 664 will move freely over a distance X₁ before it touches slide ring 666, after which fibers 625 become stretched. If the guide wire 660 is moved through the filter 640 in the distal (rightward) direction, stop 663 will move freely over a distance X₂ before it touches slide ring 665, thereafter causing fibers 625 to hang free, as there is no axial force on slide ring 666. This means that when the filter 640 has been placed in an artery, guide wire 660 can move freely in the cross-sectional area of the frame in both radial and tangential directions without exerting any forces on this frame. Further, the guide wire 660 can also move back and forth over a total distance X (where X=X₁+X₂) in the longitudinal direction relative to the filter 640 before it influences the shape or axial position of the filter 640 in the artery. Distance X can be changed by choosing the distance between fixed stops 663 and 664. If one of these stops is removed, distance X is maximized. The distal end section 662 of guide wire 660 must be long enough to prevent slide ring 665 from extending past distal end section 662 and becoming disengaged. With the construction of slide rings 665 and 666 on guide wire 660, the guide wire can be rotated around its length axis without influencing the position and shape of the filter 640 and its frame 650.

Further, the high degree of flexibility inherent in this design allows the length of frame 650 to be shortened and thus it makes the filter 640 more flexible and more easily usable in curvaceous arteries and arteries with limited space. In a highly curved artery, guide wire 660 may even touch the inner wall of frame 650 without exerting relevant forces on the filter 640. Even with a highly bent guide wire 660, the filter 640 will still maintain its full contact with the artery wall and guarantee a safe functioning of the device for a wide range of artery diameters and geometries. As can be seen from a comparison of FIG. 33 with FIGS. 31 and 32, the design of FIG. 33 gives a much smaller proximal surface of frame 650. In FIGS. 29-32, spokes 456 and the proximal side of tube 455 have a certain surface area that reduces blood flow. This surface area is significantly reduced in FIG. 33, because only a few thin fibers 625 are interposed in the blood flow. Another advantage is that debris in the blood will less likely adhere to the thin pulling fibers 625 than to the proximal side of tube 455 and spokes 456 of FIGS. 29-32. An additional treatment of pulling fibers 625 to reduce the tendency of blood cells to adhere thereto is could also be employed, and is a part of this invention as well. As previously mentioned, pulling fibers 625 may be made from the same or different material as reinforcement fibers 620. An example of such a fiber (in addition to those previously mentioned) would be a composite fiber made of a Nitinol filament core surrounded by a multifilament ultra high molecular weight highly oriented polymer. The Nitinol can be used to give some shape control to the fiber, for example to prevent adjacent fibers from becoming entangled. The polymer multifilament, besides having high strength and low strain, can have for example anti-thrombogenic or related agents embedded therein.

In FIG. 34, the filter 640 of FIG. 33 is shown in a compressed size profile, in which it is being delivered from a delivery sheath 600. Sheath 600 has a wall 606 and a distal end 607. At the proximal side of the guide wire 660, a pushing force F is applied in the distal direction, while sheath 600 is either being pulled back in the proximal direction or held in place. Stop 663 on guide wire 660 is now in direct contact with slide ring 665, and force F is transferred by this ring to the reinforcement fibers 620 of the filter membrane 610. By the resulting pulling force in the membrane 610 and fibers 620, the filter 640 is stretched. Consequently, the pulling force is transferred to the collapsed frame 650 via hinge sites 659. The frame 650 and filter 640 will easily slide out of sheath 600 by this pulling force, followed by the presently unloaded pulling fibers 625 and slide ring 666. As can be seen, the proximal section of frame 650, to which the fibers 625 are attached, is slightly tapered (bent inwards) to create a conical proximal side of frame 650. In another embodiment (not shown), the proximal section of frame 650 may be cylindrical, tapered in the reverse angle (i.e., bent outward) or have any other geometry that either makes retrieval easier or serves as an anchoring to hold the filter 640 and frame 650 in place in the artery.

FIG. 35 shows the filter 640 in a position to be retracted into a removal sheath 600, the latter of which has a wall 606 and a distal end 607. At distal end 607, the removal sheath 600 may have a flared end section 607A, as shown in FIG. 35a, a chamfered wall 607B, as shown in FIG. 35b, or a combination thereof. Distal end 607 must enable the retrieval of the filter 640 into the lumen of sheath 600 by a pulling force, which is applied to the proximal end of guide wire 660 while sheath 600 is being moved in the distal direction or is being held in place. The tapered proximal section of the frame 650 also assists its insertion into removal sheath 600. The force F₁, applied to guide wire 660, is transferred by stop 664 to slide ring 666, which distributes the force to fibers 625 that are now pulling on the proximal section attachment points 658 of the proximal section of frame 650. The ends of fibers 625 can be attached by any technique that is available, for example by putting them through respective holes in hinge sites 659 of frame 650, and securing them by a knot 685 on the inside frame surface. The holes in attachment points 658 can have several shapes, dependant on the method of attaching the fibers 625. The hole may be circular, like shown, oval or the like. If making a knot in pulling fiber 625 is not favorable, the fiber may be formed as a continuous loop, running back and forth to the slide ring 666. Attachment of such a continuous loop may even be easier if there are two slots, creating hooks on both sides of the strut end of frame 650. An example is attachment by means of a snap fit lock in the strut end. The proximal section of frame 650 have been formed in such a way that tips defining the end at the attachment points 658 are slightly curved inside with a conical top angle that is larger than the top angle of the cone defined by the stretched fibers 625, just before the proximal section enters into removal sheath 600. This is done to prevent the attachment points 658 of the frame proximal section from becoming stuck at the distal end 607 of the removal sheath 600.

With the tapered shape of frame 650, the tension force in fibers 625 will easily make it possible to slide the removal sheath 600 over the frame 650 until it is completely covered by this sheath 600. Membrane 610, eventually filled with embolic debris, does not have to be pulled into sheath 600 completely; it can instead extend from the distal end 607 while the whole device is removed from the artery.

FIG. 36a and 36b are side views of an alternative embodiment frame 750, in its expanded and collapsed shapes, respectively. This embodiment is shorter than the embodiment of FIGS. 33-35, and, in particular, lacks the distal end portion of the embodiment of FIGS. 33-35. Instead, frame 750 is composed of struts 757 configured in a zigzag-pattern. Here again the proximal section has attachment points 758 that are curved inwardly with curved tips 756 and it has attachment holes 754 for the fibers (not presently shown). The fact that the frame 750 is not subjected to a pushing force during deployment from, or retraction into, a sheath enables a further downscaling of the frame struts 757 and thus a miniaturization of the delivery profile of the device. This is also enhanced by the fact that the guide wire (not presently shown) does not influence the shape and position of the filter upon angioplasty and stenting, so the frame 750 can now also be made lighter.

Referring next to FIG. 37, another embodiment of a medical device with filter 840 frame 850 is shown. Elongated attachment parts 855 are formed at the attachment points 858 of the frame proximal section in order to bring the holes 854 for the attachment of pulling fibers 825 farther away from the expandable and collapsible unit cells of the frame 850. This increased length helps to achieve a smoother shape upon shape setting, so that struts 857 will have the desired curvature that is needed to slide easily into the removal sheath 800 (shown in FIG. 38). Placement of the attachment holes 854 at the very proximal tip of the frame struts 857 will also help to allow the frame 850 to be pulled back into the removal sheath 800 without the risk of getting stuck at the sheath entrance. The elongated struts 857 forming frame 850 can be shape set into almost any desirable angle. A part of the struts 857 may be parallel with the length axis of the filter 840, while another part or parts may be angled inside or outside, as needed for smooth removal of the device. Outside angled tips may even help to anchor the frame 850 in the blood vessel for more axial stability.

Referring next to FIG. 38, another feature of the present embodiment is shown. The design of a filter 840 according to the invention with flexible fibers 825 makes it possible to push sheath 800 over guide wire 860 until the distal end 802 of sheath 800 reaches deep into the filter 840. In this situation, sheath 800 may also function as a tube, where its positioning inside or beyond the frame 850 opens the possibility of flushing and/or suction through it in order to move debris either deeper into the distal end of the filter 840 or to suction debris out of filter 840. Flushing with certain liquids can also help to make the debris smaller. An additional treatment device can also be inserted through sheath 800 disposed inside the filter 840. This additional treatment device can be any means for inspection, measuring or all kinds of treatments like breaking up of clots by mechanical means, laser, ultrasonics, or the like. Additional retrieval devices may be brought into the filter 840 through sheath 800. The fibers 825 will easily move with distal end 802 of sheath 800 and, dependant on the length of fibers 825, the most distal position of sheath 800 can be chosen.

FIG. 39 shows another embodiment for the shape of a filter 940, with an additional reservoir 942 for storage of debris. Normally it can be expected that the major part of the debris will collect most distally, leaving the most proximal holes 930 open for blood flow. This can be improved by providing additional reservoir 942, which is connected to the conical section 943 of filter 940 by a portion 944. If the diameter of reservoir 942 is half the maximum diameter of the frame 950, the surface area that remains free for blood flow between the wall of the full reservoir and the artery wall is still 75% of the maximum surface area of the artery. The capacity of reservoir 942 can be chosen so that the closure of filter holes 930 in section 943 by abundant debris is most unlikely. Additional flushing and/or suction similar to that of the embodiment shown in FIG. 38 may also be undertaken. Continuous monitoring of the blood flow beyond the distal end of the filter 940 can be employed to provide information regarding removal of the filter 940. The shape and diameter of reservoir 942 will be dependent on the expected diameter and geometry of the artery that will be treated. The shape of reservoir 942 can be determined by reinforcement fibers 920. The membrane 910 may for example be elastic, while the fibers 920 can have a limited stretchability. Dependent on the pressure inside the reservoir 942, the diameter of the membrane 910 can be made to vary until it reaches a certain predetermined value, when the embedded fibers 920 reach their strain limit. Such fibers 920 will have a more or less tangential orientation.

A filter according to the invention, particularly because of the flexibility of the fibers 920, allows an element, such as tubular sheath 800 of FIG. 38, to penetrate into the region enclosed by the membrane 910 to apply suction to debris contained in the filter bag either continuously or intermittently. This is particularly applicable to the distal filter of a two filter assembly. The sheath 800 can be introduced over a guide wire 960 associated with the filter 940 and can enter the filter 940 with no risk of perforating it. The safety of applying suction to the interior of the filter 940 is ensured by the nature of the material used for the membrane 910 and reinforcement fibers 920. Such suction allows the filter 940 to be maintained relatively free of debris and helps to achieve a relative stability in blood flow through the membrane 910. In addition, the suction element enables the filter 940 to be kept in a relatively empty condition prior to its being closed and withdrawn and prior to the use of a distal retrieval filter.

The frames and composite structures as shown and described herein may be used not only in relation to filters, they can also be used in numerous other medical (as well as non-medical) devices. Examples include a removable temporary stent, dilator, reamer, graft housing, valve, delivery platform for drugs, radiation or gene therapy, or any other device that has to be placed and removed after some time. As will be appreciated by those skilled in the art, the application of the present invention (in all of the aforementioned configurations) is not restricted to arteries, but can be used for all body lumens or in other places in the body. In addition, it will be appreciated that in certain situations, more than a single frame may be used. Dependent on the application, the membrane is semipermeable or provided with holes for filtering function or improvement of cell ingrowth. Holes in the membrane can further be used to store drugs, which are slowly released from the membrane. Further holes can be used for attachment to surrounding frames or tissues. The hole pattern can be applied before, during or after the procedure of embedding the fibers. A further example is a thin but strong membrane that is held in shape by a frame with a different shape as shown in FIGS. 33-39. A frame does not necessarily have to be deformed before insertion, although if such deformation is desirable, it may be brought into a more suitable shape for insertion. By way of example, it can be a cylindrical expandable type, including being self-expandable. However, it may also be folded or stretched upon insertion or made deformable elastically or plastically. An example of a plastically deformable device is a surgical clip for closure of a wound or other opening. A reinforced membrane according to an embodiment of the present invention may cover such a clip to make it more leak-resistant. As previously mentioned, delivery of devices according to the invention is not restricted to the use of a guide wire in combination with a restraining sheath. Included in the invention is also delivery by any elongated member, for example a tubular catheter or a balloon catheter, a surgical tool, instrument, or even by the surgeons hands.

### Temporary devices

As previously discussed, the present invention also includes the use of pulling fibers connected to an expandable frame. The embodiments depicted in FIGS. 33-39 for the filter could also be used to allow ease of collapse of a temporary device. In FIGS. 33-39, the temporary device is always connected to the guide wire as long as the pulling fibers remain connected to the proximal slide ring. In certain circumstances, it may be necessary to disconnect the pulling wires from the frame or the guide wire. This may be the case if the decision is made that the device has to stay in the lumen for an extended period of time, or eventually becomes permanent. Examples of temporary devices which may or may not be released according to the invention include filters, stents, valves, baskets, membrane-covered clips, reamers, dilators, delivery platforms for drugs, radiation treatment or the like.

Such a remotely controlled detachment from the guide wire can be done in several ways. One example is that the pulling fibers are disconnected from the slide ring. This can be done by remote changing of the shape of the slide ring, thus unclamping the pulling fibers from this slide ring. Another possibility is that each pulling fiber has an eyelet at the proximal end, and all these eyelets are connected with a single long fiber, which runs through these eyelets and of which at least one end can be held or released by the operator. If one free end of this long fiber is released, it will slide through all eyelets, thus disconnecting the strut fibers from the guide wire. In another embodiment the fibers can be disconnected by cutting, melting or breaking.

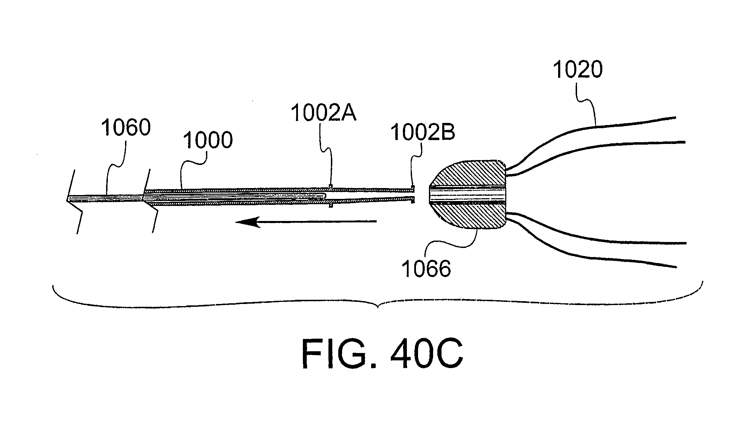

Referring next to FIGS. 40a through 40c, a further possibility of detaching a device from a guide wire is shown, where the fibers remain connected to a ring, but the ring itself is detached from the guide wire. Referring with particularity to FIG. 40a, a simple release mechanism is made from a deformable tube 1000, where the tube 1000 fits in ring 1066. Tube 1000 is mounted to guide wire 1060. Distal end 1002 of tube 1000 is provided with two stops 1002A and 1002B, one configured to engage a proximal side of ring 1066, and one to engage the ring's distal side. In this configuration, the stops 1002A, 1002B function as a lock for the distal end of tube 1000. Ring 1066 is connected to expandable frame 1050 by means of struts or fibers 1020. The distal end 1002 is provided with length slots 1002C, which enable a local diameter change of tube 1000 at its distal end 1002. The deformation of distal end 1002 can be elastic or plastic. In addition, distal end 1002 can for example be made from nitinol and be heat treated to have a reduced diameter in its unstrained state. The guide wire 1060, if located in distal end 1002, keeps it in a cylindrical shape, thus pushing stops 1002A and 1002B outward in such a way that axial movement of tube 1000 causes movement of ring 1066. This is clearly shown in FIG. 40a, where ring 1066 is firmly attached to distal end 1002 of tube 1000. Referring with particularity to FIG. 40b, removal of guide wire 1060 relative to tube 1000 allows tube 1000 to deform to a smaller diameter, until stops 1002A, 1002B bend enough inward to lose contact with ring 1066. Referring with particularity to FIG. 40c, guide wire 1060 and tube 1000 are completely detached from ring 1066 and thus from fibers 1020 and frame 1050 (the latter shown in FIG. 40a). An alternative for the external stops 1002A and 1002B can be an elastic pin (not shown) which pushes through a side hole (not shown) in the tube 1000 at the location where ring 1066 is mounted. The elastic pin only grips ring 1066 as long as the pin is pushed outward by central wire 1060. The remainder of the apparatus works the same as described above.

In the situation that a device has two slide rings mounted on the same guide wire, like the filter of FIGS. 33-39, the release mechanism of FIGS. 40a-40c may first be used to place the device while the end of tube 1000 is in contact with the most distal ring. After releasing this distal ring, guide wire 1060 can be pushed into distal section 1002 of tube 1000 to ensure a good grip on the proximal ring while guide wire 1060 is pulled back. If release of the proximal ring is necessary, the procedure of pulling back of guide wire 1060 is repeated. In such an approach, several stages of gripping and release are possible with a single coupling tool and a series of sliding rings.

## Claims

1. A medical device configured to be disposed within a body lumen (50, 58, 64, 66; 80, 82, 90, 92) suitable for use as a vascular filter, said device comprising:
a thin sheet-like membrane (410; 510; 610; 910) that is comprised of a polymer, an organic tissue, or a tissue of human or animal origin, said membrane defining a plurality of holes (430; 530; 630; 930) each one of said plurality of holes being in the range of 50 microns to 300 microns in diameter, said plurality of holes acting so to allow passage of a body fluid though the membrane, while preventing passage of particles above a certain size; and
reinforcement fibers (420; 520; 620; 920) embedded in said membrane to form a composite structure (440; 540; 640; 840; 940) therefrom; and
a frame (450; 550; 650; 750; 850; 950; 1050) attached to said composite structure to hold said membrane in a desired shape and constructed so as to collapse and expand said composite structure, said frame comprising a proximal end and a distal end, wherein said reinforcement fibers are attached to said frame.

2. The device of claim 1, further comprising an elongated member (460; 560; 660; 860; 960; 1060) configured to transport said device to an appropriate location in said body lumen (50, 58, 64, 66; 80, 82, 90, 92).

3. The device of claim 2, wherein said elongated member comprises a guide wire (2; 12; 306; 460; 560; 660; 860; 960; 1060) attached to at least one of said frame (450; 550; 650; 750; 850; 950; 1050) or said composite structure (440; 540; 640; 840; 940).

4. The device of claim 3, wherein said proximal end of said frame (450; 550; 650; 750; 850; 950; 1050) is remote from said membrane unit.

5. The device of claim 2, further comprising pulling fibers (625; 825; 1020) connecting said proximal end of said frame (450; 550; 650; 750; 850; 950; 1050) to said guide wire (460; 560; 660; 860; 960; 1060) to enable said device to be retracted into a removal sheath (600; 800; 1000) by a pulling force on said guide wire in order to retrieve said device from said body lumen (50; 58; 64; 66; 80; 82; 90; 92).

6. The device of claim 2, wherein said distal end of said frame (450; 550; 650; 750; 850; 950; 1050) is adjacent said membrane unit, and wherein said reinforcement fibers (420; 520; 620; 920) are connected between said distal end of said frame and said guide wire (460; 560; 660; 860; 960; 1060) for enabling said device to be extracted from a delivery sheath (600; 800; 1000) by pushing on said guide wire to impose a pulling force on said composite structure (440; 540; 640; 840; 940) in order to introduce said device into said body lumen (50; 58; 64; 66; 80; 82; 90; 92).

7. The device of claim 2, further comprising a plurality of slide rings (665, 666; 1066), each of said slide rings connected to opposing ends of said device such that said slide rings are responsive to displacement forces imparted thereto by said guide wire (460; 560; 660; 860; 960; 1060).

8. The device of claim 7, wherein said reinforcement fibers (420; 520; 620; 920) are directly attached to one of said slide rings (665; 1066) and said distal end of said frame (450; 550; 650; 750; 850; 1050).

9. The device of claim 8, further comprising pulling fibers (625; 825; 1020) connecting said proximal end of said frame to said guide wire for enabling said device to be retracted into a removal sheath (600; 800;1000) by a pulling force on said guide wire in order to retrieve said device from said body lumen.

10. The device of claim 8, wherein said proximal section of said frame (450; 550; 650; 750; 850; 1050) is tapered to facilitate retraction of said frame into a sheath (600; 800;1000).

11. The device of claim 3, further comprising a pressure sensing tip coupled to said guide wire (2; 12; 306; 460; 560; 660; 860; 960 ;1060).

12. The device of claim 3, wherein said frame (450; 550; 650; 750; 850; 1050) is configured to allow said guide wire (2; 12; 306; 460; 560; 660; 860; 960 ;1060) to move freely in axial, radial, tangential and rotational directions within said frame when said frame is in an expanded state without influencing the position and shape of said device.

13. The device of claim 1, wherein said device is configured as one filter of a double filter system.

14. The device of claim 3, wherein said frame (450; 550; 650; 750; 850; 1050) has elongated struts (26; 36; 48; 457; 457; 557; 757; 857) that define attachment points (658; 758; 858) at said proximal end to facilitate connection of said frame to said guide wire.

15. The device of claim 14, further comprising pulling fibers (625; 825; 1020) connected to said attachment points (658; 758; 858) by means of attachment holes (754; 854) disposed therein.

16. The device of claim 14, further comprising pulling fibers (625; 825; 1020) connected to said attachment points (754; 854) by gluing or welding.

17. The device of claim 3, further comprising a hollow tube (800; 1000) advanceable into a region at least partially enclosed by said composite structure (440; 540; 640; 840; 940) when said composite structure is in an open state.

18. The device of claim 17, wherein said guide wire (2; 12; 306; 460; 560; 660; 860; 960 ;1060) is configured to fit within said hollow tube (800; 1000).

19. The device of claim 17, wherein said tube (800; 1000) is configured to perform at least one of a suction, flushing, inspection, measuring, clot-breaking, and retrieval device introduction functions while said tube is advanced into said at least partially enclosed region.

20. The device of claim 19, wherein said hollow tube (800; 1000) is dimensioned to serve as a removal sheath for said device.

21. The device of claim 2, wherein said elongated member (460; 560; 660; 860; 960; 1060) is configured to be detached from said frame (450; 550; 650; 750; 850; 950; 1050).

22. The device of claim 21, further comprising:
a slide ring (665, 666; 1066) coupled to said device; and
a hollow tube disposed within said slide ring, wherein said hollow tube comprises a longitudinally split distal end (1002) movably responsive to said elongated member (460; 560; 660; 860; 960; 1060) such that when said elongated member is adjacent said longitudinally split distal end, said longitudinally split distal end expands, thereby affixing said hollow tube to said slide ring, and when said elongated member is removed from said longitudinally split distal end, said longitudinally split distal end contracts, thereby allowing said hollow tube to be removed from said slide ring such that said device remains in said body lumen even after said hollow tube is removed.

23. The device of claim 1, wherein said reinforcement fibers (420; 520; 620; 920) are attached to said frame (450; 550; 650; 750; 850; 950; 1050) by connections that are configured to form hinges (459; 559; 659) at the place of attachment to the frame.

24. The device of claim 1, wherein said plurality of holes (430; 530; 630; 930) are arranged in a substantially repeating pattern in said membrane (410; 510; 610; 910).

25. The device of claim 1, wherein the material making up said reinforcement fibers (420; 520; 620; 920) have a tensile stress and modulus of elasticity greater than the material making up said membrane (410; 510; 610; 910).

26. The device of claim 1, wherein said reinforcement fibers (420; 520; 620; 920) comprise a plurality of different fiber types, including fibers for shape control combined with fibers with high tensile strength.

27. The device of claim 1, wherein said reinforcement fibers (420; 520; 620; 920) are monofilament or multi-filament fibers.

28. The device of claim 26, wherein said reinforcement fibers (420; 520; 620; 920) are discontinuous and dispersed throughout said membrane (410; 510; 610; 910).

29. The device of claim 1, wherein said reinforcement fibers (420; 520; 620; 920) are coated with a polymer to enhance adhesion between said reinforcement fibers and said membrane (410; 510; 610; 910).

30. The device of claim 1, wherein said reinforcement fibers (420; 520; 620; 920) are glued to said membrane (410; 510; 610; 910).

31. The device of claim 1, wherein the material making up said reinforcement fiber (420; 520; 620; 920) is selected from the group consisting of carbon, glass, ceramics, metals, metal alloys, polymers and combinations thereof.

32. The device of claim 1, further comprising a biocompatible material disposed on at least a portion of said device.

33. The device of claim 32, wherein said biocompatible material prevents the adherence of emboli or platelets to said device.

34. The device of claim 32, wherein said biocompatible material releases a drug into said body lumen.

35. The device of claim 1, wherein said composite structure (440; 540; 640; 840; 940) is a filter that is expandable into an expanded state, said filter comprising a substantially closed distal end and an open proximal end such that said filter tapers from said proximal end to said distal end.

36. The device of claim 35, further comprising a reservoir (942) in said filter (440; 540; 640; 840; 940) that extends from said distal end, said reservoir defining a debris storage space.

37. The device of claim 1, wherein said frame (450; 550; 650; 750; 850; 950; 1050) allows said device to expand until a predetermined expanded size limit is reached.

38. The device of claim 35, wherein said reinforcement fibers (420; 520; 620; 920) are oriented in such a way as to give said composite structure (440; 540; 840; 940) a shape that depends, within predetermined limits, on the pressure difference across said composite structure.

39. The device of claim 1, wherein said device comprises a removable temporary stent, a dilator, a reamer, a graft housing, a valve, a surgical clip or a delivery platform for drugs, radiation or gene therapy.

40. The device of claim 3, wherein said frame (450; 550; 650; 750; 850; 950; 1050) is expandable such that in a first state, said frame and said composite structure (440; 540; 640; 840; 940) define a first size profile that is configured to be transported by said elongated member (460; 560; 660; 860; 960 ;1060) to a desired location within said body lumen (50; 58; 64; 66; 80; 82; 90; 92), while in a second state, said frame and said composite structure define a second size profile that is configured to engage said body lumen.

41. The device of claim 40, further comprising:
a first ring (665) slidably disposed on said elongated member (460; 560; 660; 860; 960; 1060) and coupled to a distal end of said composite structure (440; 540; 640; 840; 940);
a second ring (666; 1066) slidably disposed on said elongated member and coupled to a proximal end of said frame (450; 550; 650; 750; 850; 950; 1050); and
a plurality of stops (663, 664; 1002A, 1002B) affixed to the guide wire (2; 12; 306; 460; 560; 660; 860; 960; 1060) such that upon contact between one of said stops and one of said first or second rings due to movement of said elongated member, said device moves either into or out of said body lumen.

42. The device of claim 40, wherein said elongated member (460; 560; 660; 860; 960; 1060) comprises a guide wire.

43. The medical device of claim 1, wherein said composite structure (440; 540; 640; 840; 940) comprises:
a membrane (410; 510; 610; 910); and
first fibers (420; 520; 620; 920) coupled to said membrane to form said composite structure;
a frame (450; 550; 650; 750; 850; 950; 1050) attached to said composite structure;
second fibers (625; 825; 1020) coupled to said frame;
a guide wire (2; 12; 306; 460; 560; 660; 860; 960; 1060) coupled to said frame and said composite structure through said first and second fibers such that said first and second fibers and said guide wire are configured to move said composite structure and said frame.

44. The device of claim 43, wherein said first fibers comprise reinforcement fibers (420; 520; 620; 920).

45. The device of claim 43, wherein the material making up said first (420; 520; 620; 920) and second (625; 825; 1020) fibers is the same.

46. The device of claim 44, wherein said reinforcement fibers (420; 520; 620; 920) are discontinuous and dispersed throughout said membrane (410; 510; 610; 910).

47. A method of fabricating a medical device configured to be disposed within a body lumen (50, 58, 64, 66; 80, 82, 90, 92), suitable for use as a vascular filter, said method comprising:
providing a removable mold (401) in substantially a shape of said device;
covering said mold with membrane (410) material, the membrane material comprising a thin sheet-like membrane that is comprised of a polymer, an organic tissue, or a tissue of human or animal origin;
placing fibers (420) in contact with said membrane (410) material;
covering said fibers (420) with additional membrane material to form a composite structure (440) comprising a sheet membrane having reinforcement fibers embedded therein;
connecting a frame (450) to said composite structure (440), wherein said step of connecting said frame (450) to said composite structure (440) comprises attaching said reinforcement fibers (420) disposed in said composite structure to said frame;
forming a plurality of holes (430; 530; 630; 930) in said membrane, each one of said plurality of holes being in the range of 50 microns to 300 microns in diameter; and
removing said mold.

48. The method of claim 47, comprising the additional step of covering said mold with an intermediate material that is easily separated from said membrane (410) material prior to said step of covering said mold (401) with said membrane material.

49. The method of claim 48, comprising the additional step of removing said intermediate material from said membrane (410).

50. The method of claim 47, wherein said step of removing the mold (401) is by melting, dissolving, or deforming the mold.

51. The method of claim 50, wherein the mold (401) is made of a material that dissolves in a liquid.

52. The method of claim 47, wherein the mold (401) is made of a sheath filled with fine solid grains and then vacuum sealed.

53. The method of claim 47, wherein the mold (401) is an expandable or inflatable structure.

54. The method of claim 47, further comprising coating said composite structure (440) with an additional material having a property not possessed by the materials making up said composite structure (440).

55. The method of claim 47, wherein said frame (450) is expandable.

56. The method of claim 47, comprising the additional step of connecting a guide wire (460) to at least one of said frame (450) or composite structure (440).

57. The method of claim 47, wherein said step of forming holes (430) is by laser drilling.

## Patentansprüche

1. Medizinische Vorrichtung, die dazu eingerichtet ist, in einem Körperlumen (50, 58, 64, 66; 80, 82, 90, 92) angeordnet zu sein, und sich zur Verwendung als vaskulärer Filter eignet, wobei die Vorrichtung umfasst:
eine dünne flächenkörperartige Membran (410; 510; 610; 910), die aus einem Polymer, einem organischen Gewebe oder einem Gewebe menschlichen oder tierischen Ursprungs besteht, wobei die Membran mehrere Öffnungen (430; 530; 630; 930) definiert, wobei jede der mehreren Öffnungen vom Durchmesser her im Bereich von 50 Mikron bis 300 Mikron liegt, wobei die mehreren Öffnungen so wirken, dass ein Durchtritt eines Körperfluids durch die Membran zugelassen und dabei ein Durchtritt von Partikeln über einer bestimmten Größe verhindert wird; und
Verstärkungsfasern (420; 520; 620; 920), die in die Membran eingebettet sind, um daraus eine Verbundstruktur (440; 540; 640; 840; 940) zu bilden; und
einen Rahmen (450; 550; 650; 750; 850; 950; 1050), der an der Verbundstruktur angebracht ist, um die Membran in einer gewünschten Form zu halten, und so gebaut ist, dass er die Verbundstruktur zusammenklappt und aufweitet, wobei der Rahmen ein proximales Ende und ein distales Ende umfasst, wobei die Verstärkungsfasern am Rahmen festgemacht sind.

2. Vorrichtung nach Anspruch 1, darüber hinaus ein langgestrecktes Teil (460; 560; 660; 860; 960; 1060) umfassend, das dazu eingerichtet ist, die Vorrichtung zu einer geeigneten Stelle im Körperlumen (50, 58, 64, 66; 80, 82, 90, 92) zu transportieren.

3. Vorrichtung nach Anspruch 2, wobei das langgestreckte Teil einen Führungsdraht (2; 12; 306; 460; 560; 660; 860; 960; 1060) umfasst, der am Rahmen (450; 550; 650; 750; 850; 950; 1050) und/oder an der Verbundstruktur (440; 540; 640; 840; 940) angebracht ist.

4. Vorrichtung nach Anspruch 3, wobei das proximale Ende des Rahmens (450; 550; 650; 750; 850; 950; 1050) sich fern von der Membraneinheit befindet.

5. Vorrichtung nach Anspruch 2, darüber hinaus Zugfasern (625; 825; 1020) umfassend, die das proximale Ende des Rahmens (450; 550; 650; 750; 850; 950; 1050) mit dem Führungsdraht (460; 560; 660; 860; 960; 1060) verbinden, um zu ermöglichen, dass die Vorrichtung durch eine auf den Führungsdraht wirkende Zugkraft in eine Entnahmehülse (600; 800; 1000) zurückgezogen werden kann, um die Vorrichtung aus dem Körperlumen (50; 58; 64; 66; 80; 82; 90; 92) zurückzuholen.

6. Vorrichtung nach Anspruch 2, wobei das distale Ende des Rahmens (450; 550; 650; 750; 850; 950; 1050) an die Membraneinheit angrenzt, und wobei die Verstärkungsfasern (420; 520; 620; 920) zwischen dem distalen Ende des Rahmens und dem Führungsdraht (460; 560; 660; 860; 960; 1060) angeschlossen sind, um zu ermöglichen, dass die Vorrichtung aus einer Abgabehülse (600; 800; 1000) entnommen werden kann, indem der Führungsdraht mit Schub beaufschlagt wird, um eine Zugkraft auf die Verbundstruktur (440; 540; 640; 840; 940) auszuüben, um die Vorrichtung in das Körperlumen (50; 58; 64; 66; 80; 82; 90; 92) einzuführen.

7. Vorrichtung nach Anspruch 2, darüber hinaus mehrere Gleitringe (665; 666; 1066) umfassend, wobei jeder der Gleitringe mit entgegengesetzten Enden der Vorrichtung verbunden ist, so dass die Gleitringe auf Verlagerungskräfte ansprechen, die ihnen durch den Führungsdraht (460; 560; 660; 860; 960; 1060) auferlegt werden.

8. Vorrichtung nach Anspruch 7, wobei die Verstärkungsfasern (420; 520; 620; 920) direkt an einem der Gleitringe (665; 1066) und dem distalen Ende des Rahmens (450; 550; 650; 750; 850; 1050) angebracht sind.

9. Vorrichtung nach Anspruch 8, darüber hinaus Zugfasern (625; 825; 1020) umfassend, die das proximale Ende des Rahmens mit dem Führungsdraht verbinden, um zu ermöglichen, dass die Vorrichtung durch eine auf den Führungsdraht wirkende Zugkraft in eine Entnahmehülse (600; 800; 1000) zurückgezogen werden kann, um die Vorrichtung aus dem Körperlumen zurückzuholen.

10. Vorrichtung nach Anspruch 8, wobei der proximale Abschnitt des Rahmens (450; 550; 650; 750; 850; 1050) verjüngt ist, um das Zurückziehen des Rahmens in eine Hülse (600; 800; 1000) zu erleichtern.

11. Vorrichtung nach Anspruch 3, darüber hinaus eine Druckabtastspitze umfassend, die an den Führungsdraht (2; 12; 306; 460; 560; 660; 860; 960; 1060) angeschlossen ist.

12. Vorrichtung nach Anspruch 3, wobei der Rahmen (450; 550; 650; 750; 850; 1050) dazu eingerichtet ist, den Führungsdraht (2; 12; 306; 460; 560; 660; 860; 960; 1060), wenn sich der Rahmen in einem aufgeweiteten Zustand befindet, sich frei in axialer, radialer, tangentialer und rotatorischer Richtung in dem Rahmen bewegen zu lassen, ohne die Position und Form der Vorrichtung zu beeinflussen.

13. Vorrichtung nach Anspruch 1, wobei die Vorrichtung als ein Filter eines Doppelfiltersystems ausgelegt ist.

14. Vorrichtung nach Anspruch 3, wobei der Rahmen (450; 550; 650; 750; 850; 1050) langgestreckte Streben (26; 36; 48; 457; 557; 757; 857) hat, die Anbringungspunkte (658; 758; 858) am proximalen Ende definieren, um eine Verbindung des Rahmens mit dem Führungsdraht zu erleichtern.

15. Vorrichtung nach Anspruch 14, darüber hinaus Zugfasern (625; 825; 1020) umfassend, die mit den Anbringungspunkten (658; 758; 858) mittels darin vorgesehener Anbringungsöffnungen (754; 854) verbunden sind.

16. Vorrichtung nach Anspruch 14, darüber hinaus Zugfasern (625; 825; 1020) umfassend, die mit den Anbringungspunkten (754; 854) mittels Ankleben oder Anschweißen verbunden sind.

17. Vorrichtung nach Anspruch 3, darüber hinaus ein Hohlrohr (800; 1000) umfassend, das in einen Bereich, der zumindest teilweise von der Verbundstruktur (440; 540; 640; 840; 940) umschlossen ist, vorgeschoben werden kann, wenn sich die Verbundstruktur in einem offenen Zustand befindet.

18. Vorrichtung nach Anspruch 17, wobei der Führungsdraht (2; 12; 306; 460; 560; 660; 860; 960; 1060) dazu eingerichtet ist, in das Hohlrohr (800; 1000) zu passen.

19. Vorrichtung nach Anspruch 17, wobei das Rohr (800; 1000) dazu eingerichtet ist, Saug-, Spül-, Inspektions-, Mess-, Gerinnselzertrümmerungs- und/oder Rückholvorrichtungseinführfunktionen zu erfüllen, während das Rohr in den zumindest teilweise umschlossenen Bereich vorgeschoben ist.

20. Vorrichtung nach Anspruch 19, wobei das Hohlrohr (800; 1000) dazu dimensioniert ist, als Entnahmehülse für die Vorrichtung zu dienen.

21. Vorrichtung nach Anspruch 2, wobei das langgestreckte Teil (460; 560; 660; 860; 960; 1060) dazu eingerichtet ist, vom Rahmen (450; 550; 650; 750; 850; 950; 1050) gelöst zu werden.

22. Vorrichtung nach Anspruch 21, darüber hinaus umfassend:
einen Gleitring (665, 666; 1066), der mit der Vorrichtung verbunden ist; und
ein Hohlrohr, das in dem Gleitring angeordnet ist, wobei das Hohlrohr ein der Länge nach geteiltes distales Ende (1002) umfasst, das beweglich auf das langgestreckte Teil (460; 560; 660; 860; 960; 1060) anspricht, so dass, wenn das langgestreckte Teil sich angrenzend an das der Länge nach geteilte distale Ende befindet, sich das der Länge nach geteilte distale Ende aufweitet, wodurch das Hohlrohr am Gleitring festgesetzt wird, und, wenn das langgestreckte Teil von dem der Länge nach geteilten distalen Ende entfernt ist, sich das der Länge nach geteilte distale Ende zusammenzieht, wodurch zugelassen wird, dass das Hohlrohr aus dem Gleitring entfernt werden kann, so dass die Vorrichtung, selbst nachdem das Hohlrohr entfernt wurde, in dem Körperlumen verbleibt.

23. Vorrichtung nach Anspruch 1, wobei die Verstärkungsfasern (420; 520; 620; 920) am Rahmen (450; 550; 650; 750; 850; 950; 1050) durch Verbindungen angebracht sind, die dazu eingerichtet sind, Gelenkverbindungen (459; 559; 659) an der Anbringungsstelle am Rahmen zu bilden.

24. Vorrichtung nach Anspruch 1, wobei die mehreren Öffnungen (430; 530; 630; 930) in einem sich im Wesentlichen wiederholenden Raster in der Membran (410; 510; 610; 910) angeordnet sind.

25. Vorrichtung nach Anspruch 1, wobei das Material, das die Verstärkungsfasern (420; 520; 620; 920) ausmacht, eine Zugspannung und einen Elastizitätsmodul hat, die größer sind als das Material, das die Membran (410; 510; 610; 910) ausmacht.

26. Vorrichtung nach Anspruch 1, wobei die Verstärkungsfasern (420; 520; 620; 920) mehrere verschiedene Faserarten umfassen, die Fasern zur Formsteuerung kombiniert mit Fasern mit hoher Zugfestigkeit umfassen.

27. Vorrichtung nach Anspruch 1, wobei es sich bei den Verstärkungsfasern (420; 520; 620; 920) um Monofilament- oder Multifilamentfasern handelt.

28. Vorrichtung nach Anspruch 26, wobei die Verstärkungsfasern (420; 520; 620; 920) diskontinuierlich und über die gesamte Membran (410; 510; 610; 910) verteilt sind.

29. Vorrichtung nach Anspruch 1, wobei die Verstärkungsfasern (420; 520; 620; 920) mit einem Polymer beschichtet sind, um eine Adhäsion zwischen den Verstärkungsfasern und der Membran (410; 510; 610; 910) zu verstärken.

30. Vorrichtung nach Anspruch 1, wobei die Verstärkungsfasern (420; 520; 620; 920) an die Membran (410; 510; 610; 910) angeklebt sind.

31. Vorrichtung nach Anspruch 1, wobei das Material, das die Verstärkungsfasern (420; 520; 620; 920) ausmacht, aus der Gruppe ausgewählt ist, die aus Carbon, Glas, Keramik, Metallen, Metalllegierungen, Polymeren und Kombinationen von diesen besteht.

32. Vorrichtung nach Anspruch 1, darüber hinaus ein biokompatibles Material umfassend, das zumindest auf einem Teil der Vorrichtung vorgesehen ist.

33. Vorrichtung nach Anspruch 32, wobei das biokompatible Material das Anhaften von Pfropfen oder Blutplättchen an der Vorrichtung verhindert.

34. Vorrichtung nach Anspruch 32, wobei das biokompatible Material ein Medikament in das Körperlumen freisetzt.

35. Vorrichtung nach Anspruch 1, wobei es sich bei der Verbundstruktur (440; 540; 640; 840; 940) um einen Filter handelt, der sich zu einem aufgeweiteten Zustand aufweiten kann, wobei der Filter ein im Wesentlichen geschlossenes distales Ende und ein offenes proximales Ende aufweist, so dass sich der Filter vom proximalen Ende zum distalen Ende verjüngt.

36. Vorrichtung nach Anspruch 35, darüber hinaus einen Behälter (942) in dem Filter (440; 540; 640; 840; 940) umfassend, der sich vom distalen Ende aus erstreckt, wobei der Behälter einen Lagerraum für herausgefilterte Stoffe bildet.

37. Vorrichtung nach Anspruch 1, wobei der Rahmen (450; 550; 650; 750; 850; 950; 1050) die Vorrichtung sich aufweiten lässt, bis eine vorbestimmte Aufweitungsgrößengrenze erreicht ist.

38. Vorrichtung nach Anspruch 35, wobei die Verstärkungsfasern (420; 520; 620; 920) so ausgerichtet sind, dass sie der Verbundstruktur (440; 540; 840; 940) eine Form verleihen, die innerhalb vorbestimmter Grenzen vom Druckunterschied an der Verbundstruktur abhängt.

39. Vorrichtung nach Anspruch 1, wobei die Vorrichtung einen entfernbaren temporären Stent, einen Dilatator, eine Ausräumvorrichtung, einen Transplantatbehälter, eine Klappe, eine chirurgische Klemme oder eine Abgabeplattform für Medikamente, Strahlung oder Gentherapie umfasst.

40. Vorrichtung nach Anspruch 3, wobei der Rahmen (450; 550; 650; 750; 850; 950; 1050) so aufweitbar ist, dass der Rahmen und die Verbundstruktur (440; 540; 640; 840; 940) in einem ersten Zustand ein erstes Größenprofil definieren, das dazu eingerichtet ist, vom langgestreckten Teil (460; 560; 660; 860; 960; 1060) zu einer gewünschten Stelle im Körperlumen (50; 58; 64; 66; 80; 82; 90; 92) transportiert zu werden, während der Rahmen und die Verbundstruktur in einem zweiten Zustand ein zweites Größenprofil definieren, das dazu eingerichtet ist, das Körperlumen einzunehmen.

41. Vorrichtung nach Anspruch 40, darüber hinaus umfassend:
einen ersten Ring (665), der gleitbeweglich am langgestreckten Teil (460; 560; 660; 860; 960; 1060) vorgesehen und mit einem distalen Ende der Verbundstruktur (440; 540; 640; 840; 940) verbunden ist;
einen zweiten Ring (666; 1066), der gleitbeweglich am langgestreckten Teil vorgesehen und mit einem proximalen Ende des Rahmens (450; 550; 650; 750; 850; 950; 1050) verbunden ist; und
mehrere Anschläge (663, 664; 1002A, 1002B), die am Führungsdraht (2; 12; 306; 460; 560; 660; 860; 960; 1060) festgemacht sind, so dass bei einem von einer Bewegung des langgestreckten Teils herrührenden Kontakt zwischen einem der Anschläge und dem ersten oder zweiten Ring sich die Vorrichtung entweder in das Körperlumen hinein oder aus diesem heraus bewegt.

42. Vorrichtung nach Anspruch 40, wobei das langgestreckte Teil (460; 560; 660; 860; 960; 1060) einen Führungsdraht umfasst.

43. Medizinische Vorrichtung nach Anspruch 1, wobei die Verbundstruktur (440; 540; 640; 840; 940) umfasst:
eine Membran (410; 510; 610; 910); und
erste Fasern (420; 520; 620; 920), die mit der Membran verbunden sind, um die Verbundstruktur zu bilden;
einen Rahmen (450; 550; 650; 750; 850; 950; 1050), der an der Verbundstruktur angebracht ist;
zweite Fasern (625; 825; 1020), die mit dem Rahmen verbunden sind;
einen Führungsdraht (2; 12; 306; 460; 560; 660; 860; 960; 1060), der über die ersten und zweiten Fasern mit dem Rahmen und der Verbundstruktur verbunden ist, so dass die ersten und zweiten Fasern und der Führungsdraht dazu eingerichtet sind, die Verbundstruktur und den Rahmen zu bewegen.

44. Vorrichtung nach Anspruch 43, wobei die ersten Fasern Verstärkungsfasern (420; 520; 620; 920) umfassen.

45. Vorrichtung nach Anspruch 43, wobei es sich bei dem Material, das die ersten Fasern (420; 520; 620; 920) und zweiten Fasern (625; 825; 1020) ausmacht, um dasselbe handelt.

46. Vorrichtung nach Anspruch 44, wobei die Verstärkungsfasern (420; 520; 620; 920) diskontinuierlich und über die gesamte Membran (410; 510; 610; 910) verteilt sind.

47. Verfahren zum Herstellen einer medizinischen Vorrichtung, die dazu eingerichtet ist, in einem Körperlumen (50, 58, 64, 66; 80, 82, 90, 92) angeordnet zu sein und sich zur Verwendung als vaskulärer Filter eignet, wobei das Verfahren umfasst:
Bereitstellen einer entfernbaren Form (401) in im Wesentlichen einer Gestalt der Vorrichtung;
Bedecken der Form mit Material für eine Membran (410), wobei das Membranmaterial eine dünne flächenkörperartige Membran umfasst, die aus einem Polymer, einem organischen Gewebe oder einem Gewebe menschlichen oder tierischen Ursprungs besteht;
Versetzen von Fasern (420) in Kontakt mit dem Material für die Membran (410);
Bedecken der Fasern (420) mit zusätzlichem Membranmaterial, um eine Verbundstruktur (440) zu bilden, die eine Flächenmembran mit darin eingebetteten Verstärkungsfasern umfasst;
Verbinden eines Rahmens (450) mit der Verbundstruktur (440), wobei der Schritt des Verbindens des Rahmens (450) mit der Verbundstruktur (440) umfasst, die in der Verbundstruktur vorgesehenen Verstärkungsfasern (420) mit dem Rahmen zu verbinden;
Ausbilden mehrerer Öffnungen (430; 530; 630; 930) in der Membran, wobei jede der mehreren Öffnungen vom Durchmesser her im Bereich von 50 Mikron bis 300 Mikron liegt; und
Entfernen der Form.

48. Verfahren nach Anspruch 47, den zusätzlichen Schritt des Bedeckens der Form mit einem Zwischenmaterial, das sich mühelos von dem Material für die Membran (410) trennen lässt, vor dem Schritt des Bedeckens der Form (401) mit dem Membranmaterial umfassend.

49. Verfahren nach Anspruch 48, den zusätzlichen Schritt des Entfernens des Zwischenmaterials von der Membran (410) umfassend.

50. Verfahren nach Anspruch 47, wobei der Schritt des Entfernens der Form (401) durch Schmelzen, Auflösen oder Deformieren der Form erfolgt.

51. Verfahren nach Anspruch 50, wobei die Form (401) aus einem Material hergestellt ist, das sich in einer Flüssigkeit auflöst.

52. Verfahren nach Anspruch 47, wobei die Form (401) aus einer Hülse hergestellt ist, die mit feinen, massiven Körnern gefüllt und dann vakuumversiegelt wurde.

53. Verfahren nach Anspruch 47, wobei es sich bei der Form (401) um eine aufweitbare oder aufblasbare Struktur handelt.

54. Verfahren nach Anspruch 47, darüber hinaus umfassend, die Verbundstruktur (440) mit einem zusätzlichen Material zu beschichten, das eine Eigenschaft hat, die die Materialien, die die Verbundstruktur (440) ausmachen, nicht besitzen.

55. Verfahren nach Anspruch 47, wobei der Rahmen (450) aufweitbar ist.

56. Verfahren nach Anspruch 47, den zusätzlichen Schritt des Verbindens eines Führungsdrahts (460) mit dem Rahmen (450) und/oder der Verbundstruktur (440) umfassend.

57. Verfahren nach Anspruch 47, wobei der Schritt des Ausbildens von Öffnungen (430) durch Laserbohren erfolgt.

## Revendications

1. Dispositif médical configuré pour être disposé dans une lumière corporelle (50, 58, 64, 66 ; 80, 82, 90, 92) pouvant être utilisé comme filtre vasculaire, ledit dispositif comprenant :
une membrane mince du type feuille (410 ; 510 ; 610 ; 910) composée d'un polymère, d'un tissu organique, ou d'un tissu d'origine humaine ou animale, ladite membrane définissant une pluralité de trous (430 ; 530 ; 630 ; 930) chacun de ladite pluralité de trous étant compris dans la plage de diamètre de 50 à 300 microns, ladite pluralité de trous servant à permettre le passage d'un fluide corporel à travers la membrane, tout en empêchant le passage de particules au-dessus d'une certaine grosseur ; et
des fibres de renfort (420 ; 520 ; 620 ; 920) noyées dans ladite membrane pour former une structure composite (440 ; 540 ; 640 ; 840 ; 940) à partir de celles-ci ; et
un cadre (450 ; 550 ; 650 ; 750 ; 850 ; 950 ; 1050) fixé à ladite structure composite pour maintenir ladite membrane dans une forme souhaitée et construit de manière à contracter et dilater ladite structure composite, ledit cadre comprenant une extrémité proximale et une extrémité distale, lesdites fibres de renfort étant attachées audit cadre.

2. Dispositif selon la revendication 1, comprenant en outre un élément allongé (460 ; 560 ; 660 ; 860 ; 960 ; 1060) configuré pour transporter ledit dispositif jusqu'à un emplacement approprié dans ladite lumière corporelle (50, 58, 64, 66 ; 80, 82, 90, 92).

3. Dispositif selon la revendication 2, dans lequel ledit élément allongé comprend un fil de guidage (2 ; 12 ; 306 ; 460 ; 560 ; 660 ; 860 ; 960 ; 1060) attaché à au moins l'un dudit cadre (450 ; 550 ; 650 ; 750 ; 850 ; 950 ; 1050) ou de ladite structure composite (440 ; 540 ; 640 ; 840 ; 940).

4. Dispositif selon la revendication 3, dans lequel ladite extrémité proximale dudit cadre (450 ; 550 ; 650 ; 750 ; 850 ; 950 ; 1050) est distante de ladite unité de membrane.

5. Dispositif selon la revendication 2, comprenant en outre des fibres de traction (625 ; 825 ; 1020) reliant ladite extrémité proximale dudit cadre (450 ; 550 ; 650 ; 750 ; 850 ; 950 ; 1050) audit fil de guidage (460 ; 560 ; 660 ; 860 ; 960 ; 1060) pour permettre de rétracter ledit dispositif dans une gaine de retrait (600 ; 800 ; 1000) par une force de traction sur ledit fil de guidage afin de retirer ledit dispositif de ladite lumière corporelle (50 ; 58 ; 64 ; 66 ; 80 ; 82 ; 90 ; 92).

6. Dispositif selon la revendication 2, dans lequel ladite extrémité distale dudit cadre (450 ; 550 ; 650 ; 750 ; 850 ; 950 ; 1050) est adjacente à ladite unité de membrane, et lesdites fibres de renfort (420 ; 520 ; 620 ; 920) sont connectées entre ladite extrémité distale dudit cadre et ledit fil de guidage (460 ; 560 ; 660 ; 860 ; 960 ; 1060) pour permettre l'extraction dudit dispositif hors d'une gaine de distribution (600 ; 800 ; 1000) en poussant sur ledit fil de guidage afin d'exercer une force de traction sur ladite structure composite (440 ; 540 ; 640 ; 840 ; 940) afin d'introduire ledit dispositif dans ladite lumière corporelle (50 ; 58 ; 64 ; 66 ; 80 ; 82 ; 90 ; 92).

7. Dispositif selon la revendication 2, comprenant en outre une pluralité d'anneaux coulissants (665 ; 666 ; 1066), chacun desdits anneaux coulissants étant connecté à des extrémités opposées dudit dispositif de telle sorte que lesdits anneaux coulissants répondent à des forces de déplacement qui leur sont conférées par ledit fil de guidage (460 ; 560 ; 660 ; 860 ; 960 ; 1060).

8. Dispositif selon la revendication 7, dans lequel lesdites fibres de renfort (420 ; 520 ; 620 ; 920) sont attachées directement à l'un desdits anneaux coulissants (665 ; 1066) et de ladite extrémité distale dudit cadre (450 ; 550 ; 650 ; 750 ; 850 ; 1050).

9. Dispositif selon la revendication 8, comprenant en outre des fibres de traction (625 ; 825 ; 1020) reliant ladite extrémité proximale dudit cadre audit fil de guidage pour permettre de rétracter ledit dispositif dans une gaine de retrait (600 ; 800 ; 1000) par une force de traction sur ledit fil de guidage afin de retirer ledit dispositif de ladite lumière corporelle.

10. Dispositif selon la revendication 8, dans lequel ladite section proximale dudit cadre (450 ; 550 ; 650 ; 750 ; 850 ;1050) est conique afin de faciliter la rétraction dudit cadre dans une gaine (600 ; 800 ; 1000).

11. Dispositif selon la revendication 3, comprenant en outre une pointe de détection de pression couplée audit fil de guidage (2 ; 12 ; 306 ; 460 ; 560 ; 660 ; 860 ; 960 ; 1060).

12. Dispositif selon la revendication 3, dans lequel ledit cadre (450 ; 550 ; 650 ; 750 ; 850 ; 1050) est configuré pour permettre audit fil de guidage (2 ; 12 ; 306 ; 460 ; 560 ; 660 ; 860 ; 960 ; 1060) de se déplacer librement dans des sens axial, radial, tangentiel et rotationnel dans ledit cadre quand ledit cadre est dans un état dilaté sans influencer la position et la forme dudit dispositif.

13. Dispositif selon la revendication 1, dans lequel ledit dispositif est configuré comme un filtre d'un système à double filtre.

14. Dispositif selon la revendication 3, dans lequel ledit cadre (450 ; 550 ; 650 ; 750 ; 850 ; 1050) a des étrésillons allongés (26 ; 36 ; 48 ; 457 ; 557 ; 757 ; 857) qui définissent des points de fixation (658 ; 758 ; 858) au niveau de ladite extrémité proximale pour faciliter la connexion dudit cadre audit fil de guidage.

15. Dispositif selon la revendication 14, comprenant en outre des fibres de traction (625 ; 825 ; 1020) connectées auxdits points de fixation (658 ; 758 ; 858) au moyen de trous de fixation (754 ; 854) disposés dans ceux-ci.

16. Dispositif selon la revendication 14, comprenant en outre des fibres de traction (625 ; 825 ; 1020) connectées auxdits points de fixation (754 ; 854) par collage ou soudage.

17. Dispositif selon la revendication 3, comprenant en outre un tube creux (800 ; 1000) pouvant avancer dans une région au moins partiellement enfermée par ladite structure composite (440 ; 540 ; 640 ; 840 ; 940) quand ladite structure composite est dans un état ouvert.

18. Dispositif selon la revendication 17, dans lequel ledit fil de guidage (2 ; 12 ; 306 ; 460 ; 560 ; 660 ; 860 ; 960 ; 1060) est configuré pour entrer dans ledit tube creux (800 ; 1000).

19. Dispositif selon la revendication 17, dans lequel ledit tube (800 ; 1000) est configuré pour exécuter au moins l'une de fonctions d'aspiration, de purge, d'inspection, de mesure, de dissolution de caillot, et d'introduction de dispositif de retrait alors que ledit tube est avancé dans ladite région au moins partiellement enfermée.

20. Dispositif selon la revendication 19, dans lequel ledit tube creux (800 ; 1000) est dimensionné pour servir de gaine de retrait dudit dispositif.

21. Dispositif selon la revendication 2, dans lequel ledit élément allongé (460 ; 560 ; 660 ; 860 ; 960 ; 1060) est configuré pour être détaché dudit cadre (450 ; 550 ; 650 ; 750 ; 850 ; 950 ; 1050).

22. Dispositif selon la revendication 21, comprenant en outre :
un anneau coulissant (665, 666 ; 1066) couplé audit dispositif ; et
un tube creux disposé dans ledit anneau coulissant, ledit tube creux comprenant une extrémité distale fendue longitudinalement (1002) répondant par un déplacement audit élément allongé (460 ; 560 ; 660 ; 860 ; 960 ; 1060) de telle sorte que lorsque ledit élément allongé est adjacent à ladite extrémité distale fendue longitudinalement, ladite extrémité distale fendue longitudinalement se dilate, fixant ainsi ledit tube creux audit anneau coulissant, et quand ledit élément allongé est retiré de ladite extrémité distale fendue longitudinalement, ladite extrémité distale fendue longitudinalement se contracte, permettant ainsi de retirer ledit tube creux dudit anneau coulissant de telle sorte que ledit dispositif reste dans ladite lumière corporelle même après le retrait dudit tube creux.

23. Dispositif selon la revendication 1, dans lequel lesdites fibres de renfort (420 ; 520 ; 620 ; 920) sont attachées audit cadre (450 ; 550 ; 650 ; 750 ; 850 ; 950 ; 1050) par des connexions qui sont configurées pour former des charnières (459 ; 559 ; 659) à l'endroit de fixation au cadre.

24. Dispositif selon la revendication 1, dans lequel ladite pluralité de trous (430 ; 530 ; 630 ; 930) est agencée en une configuration sensiblement répétitive dans ladite membrane (410 ; 510 ; 610 ; 910).

25. Dispositif selon la revendication 1, dans lequel le matériau composant lesdites fibres de renfort (420 ; 520 ; 620 ; 920) a une contrainte de traction et un module d'élasticité supérieur au matériau qui compose ladite membrane (410 ; 510 ; 610 ; 910).

26. Dispositif selon la revendication 1, dans lequel lesdites fibres de renfort (420 ; 520 ; 620 ; 920) comprennent une pluralité de différents types de fibres, dont des fibres de contrôle de forme combinées à des fibres à grande résistance à la traction.

27. Dispositif selon la revendication 1, dans lequel lesdites fibres de renfort (420 ; 520 ; 620 ; 920) sont des fibres à un seul ou à plusieurs filaments.

28. Dispositif selon la revendication 26, dans lequel lesdites fibres de renfort (420 ; 520 ; 620 ; 920) sont discontinues et dispersées dans toute ladite membrane (410 ; 510 ; 610 ; 910).

29. Dispositif selon la revendication 1, dans lequel lesdites fibres de renfort (420 ; 520 ; 620 ; 920) sont revêtues d'un polymère pour rehausser l'adhérence entre lesdites fibres de renfort et ladite membrane (410 ; 510 ; 610 ; 910).

30. Dispositif selon la revendication 1, dans lequel lesdites fibres de renfort (420 ; 520 ; 620 ; 920) sont collées à ladite membrane (410 ; 510 ; 610 ; 910).

31. Dispositif selon la revendication 1, dans lequel le matériau composant lesdites fibres de renfort (420 ; 520 ; 620 ; 920) est sélectionné dans le groupe consistant en carbone, verre, céramique, métaux, alliages métalliques, polymères et leurs combinaisons.

32. Dispositif selon la revendication 1, comprenant en outre un matériau biocompatible disposé sur au moins une partie dudit dispositif.

33. Dispositif selon la revendication 32, dans lequel ledit matériau biocompatible empêche l'adhérence d'emboles ou de plaquettes sur ledit dispositif.

34. Dispositif selon la revendication 32, dans lequel ledit matériau biocompatible libère un médicament dans ladite lumière corporelle.

35. Dispositif selon la revendication 1, dans lequel ladite structure composite (440 ; 540 ; 640 ; 840 ; 940) est un filtre dilatable dans un état dilaté, ledit filtre comprenant une extrémité distale sensiblement fermée et une extrémité proximale ouverte de telle sorte que ledit filtre soit conique de ladite extrémité proximale à ladite extrémité distale.

36. Dispositif selon la revendication 35, comprenant en outre un réservoir (942) dans ledit filtre (440 ; 540 ; 640 ; 840 ; 940) qui s'étend depuis ladite extrémité distale, ledit réservoir définissant un espace de stockage de débris.

37. Dispositif selon la revendication 1, dans lequel ledit cadre (450 ; 550 ; 650 ; 750 ; 850 ; 950 ; 1050) permet au dispositif de se dilater jusqu'à une limite prédéterminée de taille dilatée.

38. Dispositif selon la revendication 35, dans lequel lesdites fibres de renfort (420 ; 520 ; 620 ; 920) sont orientées de façon à donner à ladite structure composite (440 ; 540 ; 840 ; 940) une forme qui dépend, dans des limites prédéterminées, de la différence de pression en travers de ladite structure composite.

39. Dispositif selon la revendication 1, dans lequel ledit dispositif comprend un stent temporaire amovible, un dilatateur, un élargisseur, un réceptacle de greffe, une valve, un clip chirurgical ou une plateforme de distribution de médicament, de rayonnement ou de thérapie génique.

40. Dispositif selon la revendication 3, dans lequel ledit cadre (450 ; 550 ; 650 ; 750 ; 850 ; 950 ; 1050) est dilatable de telle sorte que dans un premier état, ledit cadre et ladite structure composite (440 ; 540 ; 640 ; 840 ; 940) définissent un premier profil de taille qui est configuré pour être transporté par ledit élément allongé (460 ; 560 ; 660 ; 860 ; 960 ; 1060), jusqu'à un emplacement souhaité dans ladite lumière corporelle (50 ; 58 ; 64 ; 66 ; 80 ; 82 ; 90 ; 92), tandis que dans un second état, ledit cadre et ladite structure composite définissent un second profil de taille qui est configuré pour s'engager avec ladite lumière corporelle.

41. Dispositif selon la revendication 40, comprenant en outre :
un premier anneau (665) disposé de manière coulissante sur ledit élément allongé (460 ; 560 ; 660 ; 860 ; 960 ; 1060) et couplé à une extrémité distale de ladite structure composite (440 ; 540 ; 640 ; 840 ; 940) ;
un second anneau (666 ; 1066) disposé de manière coulissante sur ledit élément allongé et couplé à une extrémité proximale dudit cadre (450 ; 550 ; 650 ; 750 ; 850 ; 950 ; 1050) ; et
une pluralité de butées (663, 664 ; 1002A, 1002B) fixées au fil de guidage (2 ; 12 ; 306 ; 460 ; 560 ; 660 ; 860 ; 960 ; 1060) de telle sorte qu'au contact entre l'une des butées et l'un desdits premier et second anneaux en raison du mouvement dudit élément allongé, ledit dispositif entre dans ladite lumière corporelle ou en sorte.

42. Dispositif selon la revendication 40, dans lequel ledit élément allongé (460 ; 560 ; 660 ; 860 ; 960 ; 1060) comprend un fil de guidage.

43. Dispositif médical selon la revendication 1, dans lequel ladite structure composite (440 ; 540 ; 640 ; 840 ; 940) comprend :
une membrane (410 ; 510 ; 610 ; 910) ; et
des premières fibres (420 ; 520 ; 620 ; 920) couplées à ladite membrane pour former ladite structure composite ;
un cadre (450 ; 550 ; 650 ; 750 ; 850 ; 950 ; 1050) fixé à ladite structure composite ;
des secondes fibres (625 ; 825 ; 1020) couplées audit cadre ;
un fil de guidage (2 ; 12 ; 306 ; 460 ; 560 ; 660 ; 860 ; 960 ; 1060) couplé audit cadre et à ladite structure composite par le biais desdites premières et secondes fibres de telle sorte que lesdites premières et secondes fibres et ledit fil de guidage soient configurés pour déplacer ladite structure composite et ledit cadre.

44. Dispositif selon la revendication 43, dans lequel lesdites premières fibres comprennent des fibres de renfort (420 ; 520 ; 620 ; 920).

45. Dispositif selon la revendication 43, dans lequel lesdites premières (420 ; 520 ; 620 ; 920) et secondes (625 ; 825 ; 1020) fibres de renfort sont constituées du même matériau.

46. Dispositif selon la revendication 44, dans lequel lesdites fibres de renfort (420 ; 520 ; 620 ; 920) sont discontinues et dispersées dans toute ladite membrane (410 ; 510 ; 610 ; 910).

47. Procédé de fabrication d'un dispositif médical configuré pour être disposé à l'intérieur d'une lumière corporelle (50, 58, 64, 66 ; 80, 82, 90, 92), pouvant être utilisé comme filtre vasculaire, ledit procédé comprenant :
la fourniture d'un moule amovible (401) dans sensiblement une forme dudit dispositif ;
le recouvrement dudit moule avec un matériau de membrane (410), le matériau de membrane comprenant une membrane mince du type feuille composée d'un polymère, d'un tissu organique, ou d'un tissu d'origine humaine ou animale ;
le placement de fibres (420) en contact avec ledit matériau de membrane (410) ;
le recouvrement desdites fibres (420) avec un matériau de membrane supplémentaire pour former une structure composite (440) comprenant une membrane en feuille dans laquelle sont noyées des fibres de renfort ;
la connexion d'un cadre (450) à ladite structure composite (440), ladite étape de connexion dudit cadre (450) à ladite structure composite (440) comprenant la fixation desdites fibres de renfort (420) disposées dans ladite structure composite audit cadre ;
la formation d'une pluralité de trous (430 ; 530 ; 630 ; 930) dans ladite membrane, chacun de ladite pluralité de trous ayant un diamètre compris dans la plage de 50 à 300 microns ; et
le retrait dudit moule.

48. Procédé selon la revendication 47, comprenant l'étape supplémentaire de recouvrement dudit moule avec un matériau intermédiaire qui est facilement séparé dudit matériau de membrane (410) avant ladite étape de recouvrement dudit moule (401) avec ledit matériau de membrane.

49. Procédé selon la revendication 48, comprenant l'étape supplémentaire de retrait dudit matériau intermédiaire de ladite membrane (410).

50. Procédé selon la revendication 47, dans lequel ladite étape de retrait du moule (401) se fait par fusion, dissolution ou déformation du moule.

51. Procédé selon la revendication 50, dans lequel le moule (401) est réalisé en un matériau qui se dissout dans un liquide.

52. Procédé selon la revendication 47, dans lequel le moule (401) est réalisé en une gaine remplie de fins grains solides puis scellé sous vide.

53. Procédé selon la revendication 47, dans lequel le moule (401) est une structure extensible ou gonflable.

54. Procédé selon la revendication 47, comprenant en outre le revêtement de ladite structure composite (440) avec un matériau supplémentaire ayant une propriété que ne possède pas les matériaux composant ladite structure composite (440).

55. Procédé selon la revendication 47, dans lequel ledit cadre (450) est extensible.

56. Procédé selon la revendication 47, comprenant l'étape supplémentaire de connexion d'un fil de guidage (460) à au moins l'un dudit cadre (450) ou de ladite structure composite (440).

57. Procédé selon la revendication 47, dans lequel ladite étape de formation de trous (430) est réalisée par perçage au laser.
